# EUROPEAN PATENT SPECIFICATION

(11) **EP 2 611 485 B1**
(45) Date of publication and mention of the grant of the patent: **10.11.2021**
(21) Application number: 08789521.5
(22) Date of filing: 01.08.2008
(51) Int. Cl.: A61M 16/00

(54) **VENTILATOR APPARATUS AND SYSTEM FOR VENTILATION**
BELÜFTUNGSVORRICHTUNG UND BELÜFTUNGSSYSTEM
APPAREIL DE VENTILATEUR ET SYSTÈME DE VENTILATION

(30) Priority: 01.06.2007 US 924835 P; 02.06.2008 US 131922
(43) Date of publication of application: 10.07.2013
(73) Proprietor: Habashi, Nader M., Annapolis MD 21401 (US)
(72) Inventor: Habashi, Nader M., Annapolis MD 21401 (US)
(74) Representative: van der Velden, Marc
(86) International application number: PCT/IB2008/053094
(87) International publication number: WO 2008/146264

(56) References cited:
- US-A- 5 320 093
- US-A1- 2002 110 849
- US-A1- 2003 111 078
- US-A1- 2004 003 813
- US-A1- 2005 115 561
- US-A1- 2006 283 450

## Description

### BACKGROUND OF THE INVENTION

### Field of the Invention

The invention relates to the field of ventilating human patients. More particularly, the present disclosure relates to an improved ventilator and method of operation for ventilation intervention and initiation, oxygenation, recruitment, ventilation, initial weaning, airway pressure release ventilation weaning, continuous positive airway pressure weaning, and continuous and periodic management and control of the ventilator.

### Description of Related Art

The inventor herein has previously invented, among other inventions, ventilator systems and methods of operation disclosed and claimed in U.S. Patent No. 7,246,618, and in U.S. Patent Application Nos. 2008/0072901, 2006/0174884, 2003/0111 078.

Airway pressure release ventilation (APRV) is a mode of ventilation believed to offer advantages as a lung protective ventilator strategy. APRV is a form of continuous positive airway pressure (CPAP) with an intermittent release phase from a preset CPAP level. Similarly, APRV allows maintenance of substantially constant airway pressure to optimize end inspiratory pressure and lung recruitment. The CPAP level optimizes lung recruitment to prevent or limit low volume lung injury. In addition, the CPAP level provides a preset pressure limit to prevent or limit over distension and high volume lung injury.

The intermittent release from the CPAP level augments alveolar ventilation. Intermittent CPAP release accomplishes ventilation by lowering airway pressure. In contrast, conventional ventilation elevates airway pressure for tidal ventilation. Elevating airway pressure for ventilation increases lung volume towards total lung capacity (TLC), approaching or exceeding an upper inflection point of an airway pressure - volume curve (P-V curve). The P-V curve includes two limbs joined by upper and lower inflection points: an inspiratory or inspiration limb that is opposite an expiratory or expiration limb. Limiting ventilation below the upper inflection of the P-V curve is one the goals of lung protective strategies.

Subsequently, tidal volume reduction is necessary to limit the potential for over distension. Tidal volume reduction produces alveolar hypoventilation and elevated carbon dioxide levels. Reduced alveolar ventilation from tidal volume reduction has led to a strategy to increase respiratory frequency to avoid the adverse effects of hypercapnia. However, increased respiratory frequency is associated with increase lung injury. In addition, increase in respiratory frequency decreases inspiratory time and lessens the potential for recruitment. Furthermore, increasing respiratory frequency increases frequency dependency and decreases potential to perform ventilation on the expiratory limb of the P-V curve.

During APRV, ventilation occurs on the expiratory limb of a pressure - volume curve. The resultant expiratory tidal volume decreases lung volume, eliminating the need to elevate end inspiratory pressure above the upper inflection point. Therefore, tidal volume reduction is unnecessary. CPAP levels can be set with the goal of optimizing recruitment without increasing the potential for over distension. Consequently, end inspiratory pressure can be limited despite more complete recruitment, while ventilation can be maintained.

APRV was developed to provide ventilator support to patients with respiratory failure. Clinical use of APRV is associated with decreased airway pressures, decreased dead space ventilation and lower intra-pulmonary shunting as compared to conventional volume and pressure cycled ventilation. APRV limits excessive distension of lung units, thereby decreasing the potential for ventilator induced lung injury (VILI), a form of lung stress. In addition, APRV reduces minute ventilation requirements, allows spontaneous breathing efforts and improves cardiac output.

APRV is a form of positive pressure ventilation that augments alveolar ventilation and lowers peak airway pressure. Published data on APRV has documented airway pressure reduction on the order of 30 to 40 percent over conventional volume and pressure cycled ventilation during experimental and clinical studies. Such reduction of airway pressure may reduce the risk of VILI. APRV improves ventilation to perfusion ratio (V/Q) matching and reduces shunt fraction compared to conventional ventilation. Studies performed utilizing multiple inert gas dilution and excretion technique (MIGET) have demonstrated less shunt fraction, and dead space ventilation. Such studies suggest that APRV is associated with more uniform distribution of inspired gas and less dead space ventilation than conventional positive pressure ventilation.

APRV is associated with reduction or elimination of sedative, inotropic and neuromuscular blocking agents. APRV has also been associated with improved hemodynamics. In a ten year review of APRV, Calzia reported no adverse hemodynamic effects. Several studies have documented improved cardiac output, blood pressure and oxygen delivery. Consideration of APRV as an alternative to pharmacological or fluid therapy in the hemodynamically-compromised, mechanically-ventilated patient has been recommended in several case reports.

APRV is a spontaneous breathing mode of ventilation that allows unrestricted breathing effort at any time during the ventilator cycle. Spontaneous breathing in Acute Lung Injury/Acute Respiratory Distress Syndrome (ALI/ARDS) has been associated with improved ventilation and perfusion, decreased dead space ventilation and improved cardiac output and oxygen delivery. ALI/ARDS is a pathological condition characterized by marked increase in respiratory elastance and resistance.

However, most patients with ALI/ARDS exhibit expiratory flow limitations. Expiratory flow limitations results in dynamic hyperinflation and intrinsic positive end expiratory pressure (PEEP) development. In addition, ARDS patients experience increased flow resistance from external ventilator valving and gas flow path circuitry including the endotracheal tube and the external application of PEEP.

Several mechanisms can induce expiratory flow limitations in ALI/ARDS. In ALI/ARDS both functional reserve capacity (FRC) and expiratory flow reserve are reduced. Pulmonary edema development and superimposed pressure result in increased airway closing volume and trapped volume. In addition, the reduced number of functional lung units (derecruited lung units or alveolar and enhanced airway closure) decrease expiratory flow reserve further. Low volume ventilation promotes small airway closure and gas trapping. In addition elevated levels of PEEP increase expiratory flow resistance. In addition to downstream resistance, maximal expiratory flow depends on lung volume. The elastic recoil pressure stored in the preceding lung inflation determines the rate of passive lung deflation.

APRV expiratory flow is enhanced by utilization of an open breathing system and use of low (0-5 cm H₂O) end expiratory pressure. Ventilation on the expiratory limb of the P-V curve allows lower PEEP levels to prevent airway closure. Lower PEEP levels result when PEEP is utilized to prevent derecruitment rather than attempting partial recruitment. Increasing PEEP levels increases expiratory resistance, and conversely lower PEEP levels reduce expiratory resistance, thereby accelerating expiratory flow rates. Sustained inflation results in increased lung recruitment (increased functional lung units and increased recoil pressure) and ventilation along the expiratory limb (reduced PEEP and expiratory flow resistance), improving expiratory flow reserve.

In addition, release from a sustained high lung volume increases stored energy and recoil potential, further accelerating expiratory flow rates. Unlike low volume ventilation, release from a high lung volume increases airway caliber and reduces downstream resistance. Maintenance of end expiratory lung volume (EELV) to the inflection point of the flow volume curve and the use of an open system allows reduction in circuitry flow resistance. EELV is maintained by limiting the release time and titrated to the inflection point of the flow time curve.

Reduced levels of end expiratory pressure are required when ventilation occurs on the expiratory limb of the P-V curve. In ALI/ARDS, increased capillary permeability results in lung edema. Exudation from the intravascular space accumulates, and superimposed pressure on dependent lung regions increases and compresses airspaces. Dependent airspaces collapse and compressive atelectasis results in severe V/Q mismatching and shunting. Regional transpulmonary pressure gradients that exist in the normal lung are exaggerated during the edematous phase of ALI/ARDS. Patients typically being in the supine position, such that forces directed dorsally and cephalad progressively increase pleural pressures in dependent lung regions. Ventilation decreases as pleural pressure surrounding the dependent regions lowers transalveolar pressure differentials. Full ventilatory support during controlled ventilation promotes formation of dependent atelectasis, increase V/Q mismatching and intrapulmonary shunting. Increasing airway pressure can re-establish dependent transpulmonary pressure differential but at the risk of over distension of nondependent lung units.

Alternatively, spontaneous breathing, as with APRV, can increase dependent transpulmonary pressure differentials without increasing airway pressure. APRV allows unrestricted spontaneous breathing during any phase of the mechanical ventilator cycle. As noted, spontaneous breathing can lower pleural pressure, thereby increasing dependent transpulmonary pressure gradients without additional airway pressure. Increasing dependent transpulmonary pressure gradients improves recruitment and decreases V/Q mismatching and shunt.

As compared to pressure support ventilation (PSV) multiple inert gas dilution technique, APRV provides spontaneous breathing and improved V/Q matching, intrapulmonary shunting and dead space. In addition, APRV with spontaneous breathing increased cardiac output. However, spontaneous breathing during pressure support ventilation was not associated with improved V/Q matching in the dependent lung units. PSV required significant increases in pressure support levels (airway pressure) to match the same minute ventilation.

Conventional lung protective strategies are associated with increased use of sedative agents and neuromuscular blocking agents (NMBA). The increased use of sedative and NMBA may increase the time the patient must remain on mechanical ventilation ("vent days") and increase complications. NMBA are associated with prolonged paralysis and potential for nosocomial pneumonia. APRV is a form of CPAP that allows unrestricted spontaneous breathing.

Decreased usage of sedation and neuromuscular blocking agents (NMBA) has been reported with APRV. In some institutions, APRV has nearly eliminated the use of NMBA, resulting in a significant reduction in drug costs. In addition to drug cost reduction, elimination of NMBA is thought to reduce the likelihood of associated complications such as prolonged paralysis and may facilitate weaning from mechanical ventilation.

Mechanical ventilation remains the mainstay management for acute respiratory failure. However, recent studies suggest that mechanical ventilation may produce, sustain or increase the risk of acute lung injury (ALI). Ventilator induced lung injury (VILI) is a form of lung stress failure associated with mechanical ventilation and acute lung injury. Animal data suggest that lung stress failure from VILI may result from high or low volume ventilation. High volume stress failure is a type of stretch injury, resulting from over distension of airspaces. In contrast, shear force stress from repetitive airway closure during the tidal cycle from mechanical ventilation results in low volume lung injury.

Initially, lung protective strategy focused on low tidal volume ventilation to limit excessive distension and VILI. Amato in 1995 and in 1998 utilized lung protective strategy based on the pressure-volume (P-V) curve of the respiratory system. Low tidal volumes (6 ml/kg) confined ventilation between the upper and lower inflection points of the P-V curve. End expiratory lung volume was maintained by setting PEEP levels to 2 cm H₂O above the lower inflection point. Amato demonstrated improved survival and increased ventilator free days.

However, subsequent studies by Stewart and Brower were unable to demonstrate improved survival or ventilator free days utilizing low tidal volume ventilation strategy. Unlike Stewart and Brower, Amato utilized elevated end expiratory pressure in addition to tidal volume reduction. Such important differences between these studies limited conclusions as to the effectiveness of low tidal ventilation limiting ventilator associated lung injury (VALI).

Recent completion of the large controlled randomized ARDSNet trial documented improved survival and ventilator free days utilizing low tidal volume ventilation (6 ml/kg) vs. traditional tidal volume ventilation (12 ml/kg). Although the low tidal volume group (6 ml/kg) and traditional tidal volume group (12 ml/kg) utilized identical PEEP/ FiO₂ scales, PEEP levels were significantly higher in the low tidal volume group. Higher PEEP levels were required in the low tidal volume group in order to meet oxygenation goals of the study. Despite improved survival in the low tidal volume group (6 ml/kg) over traditional tidal volume group (12 ml/kg), survival was higher in the Amato study. The ARDSNet trial also failed to demonstrate any difference in the incidence of barotrauma. The higher PEEP requirements and the potential for significant intrinsic PEEP from higher respiratory frequency in the lower tidal volume group, may have obscured potential contribution of elevated end expiratory pressure on survival. Further studies are contemplated to address the issue of elevated end expiratory pressure.

In the prior art, utilization of the quasi-static inspiratory pressure versus volume (P-V) curve has been advocated as the basis or controlling a ventilator to carry out mechanical ventilation. The shape of the inspiratory P-V curve is sigmoidal and is described as having three segments. The curve forms an upward concavity at low inflation pressure and a downward concavity at higher inflation pressures. Between the lower concavity and the upper concavity is the "linear" portion of the curve. The pressure point resulting in rapid transition to the linear portion of the curve has been termed the "lower inflection point".

The lower inflection point is thought to represent recruitment of atelectatic alveolar units. The increasing slope of the P-V curve above lower inflection point reflects alveolar compliance. Above the inflection point, the majority of air spaces are opened or "recruited". Utilizing the lower inflection point of the inspiratory P-V curve plus 2 cm H₂O has been proposed to optimize alveolar recruitment. Optimizing lung recruitment prevents tidal recruitment/derecruitment and cyclic airway closure at end expiration. Ultimately, optimizing lung recruitment could potentially reduce shear force generation and low volume lung injury.

Despite an increase in the knowledge of those skilled in the relevant arts as to how to improve and maintain recruitment which minimizes the possibility of VILI and other anomalies, the systems, devices, and methods of the prior remain difficult to operate and employ for use with the best practice protocols. While due to many constraints, the often-cited challenges complained of by those skilled and practicing in the intensive care respiratory technical field is that a more automated and more accurate means is needed for applying the best practice APRV techniques.

More specifically, what has long been needed are improved devices and modes of operation that enable a clinician to more readily configure and reconfigure the desired APRV approach to respond to the individual presentation of each patient. Preferably, such improved APRV devices and methods for use would establish the capability to give the clinical practitioner a comprehensive starting point of suitable APRV parameters that could be quickly fine-tuned to meet the needs of a particular patient.

More preferably, such an improved method and ventilator device would also be able to capture the real-time patient condition information used by a clinician in monitoring patient response to the initial APRV parameters, and to generate a more automated feedback loop that would enable the improved method or device to be automatically reconfigured within clinically preferred operational and protocol constraints. Even more preferably, a new method and apparatus is needed that would also enable more accurate and smaller adjustments to the various APRV parameters that is presently possible with the present day equipment and methods, which must be manually adjusted. Such manual adjustments often result in unfavorable patient response that results from inaccurate adjustments or adjustments that cannot be made with enough precision due to the constraints or limited capabilities of the presently available equipment.

Most preferably, what is needed is a new and improved ventilator that incorporates new features and a mode of operation enabling greater flexibility, higher accuracy, and faster clinical response times in making adjustments to the various P-V curve and related parameters to accommodate unexpectedly changing patient conditions.

US 2005/11561 A1 discloses systems and methods involving an implantable device configured to perform at least one cardiac-related function, a patient-externa! respiratory therapy device, and a communication channel configured to facilitate communication between the implantable device and the respiratory therapy device. The implantable and respiratory therapy devices operate cooperatively via the communication channel to provide one or more of patient monitoring, diagnosis, and therapy. The communication channel may be configured to facilitate communication between an external processing system and at least one of the implantable device and the respiratory therapy device. The processing system is communicatively coupled to at least one of the implantable and respiratory therapy devices via the communication channel to provide one or more of patient monitoring, diagnosis and therapy.

US 2003/111078 A1 discloses a method of adjusting and guiding ventilator parameters through the use of a process which comprises: a) placing a ventilator in airway pressure release ventilation mode, b) invasive or non-invasive methods of measuring saturation of blood and carbon dioxide levels, c) ratio of spontaneous to machine minute ventilation, d) level of sedation, e) monitoring airway pressure, flow and volume to the patient, f) calculation of parameters from the determination of airway pressure, flow and volume, and g) establishing initial settings by using empiric values based on default settings.

US 2002/110849 A1 discloses a method and an apparatus to identify alveolar opening and collapse of a lung. To automatically generate the settings of ventilator parameters in a simple and gentle way, the hemoglobin will be in oxygen saturation and/or the end tidal CO₂ concentration and/or the CO₂ output are measured and processed to detect alveolar opening and closing. From the knowledge of the corresponding airway pressures, a central processing unit may generate the settings of ventilation parameters such that gas exchange is maximal while the mechanical stress of the lung tissue is minimal.

US 2006/283450 A1 discloses a gas delivery system comprising a pressure generator, a pressure sensor, a control valve, and a processor. The pressure generator pressurizes breathable gas for delivery to a patient. The pressure sensor measures a pressure difference between the pressurized breathable gas and atmospheric pressure. The control valve is disposed downstream from the pressure generator and is constructed and arranged to control a flow rate of the pressurized breathable gas. The processor controls the control valve to bring the flow rate of the pressurized breathable gas to substantially zero while the pressure generator is operating and, when the flow rate is substantially zero, determines at least one of atmospheric pressure, an atmospheric air density, or a density correction factor based at least in part on the pressure difference between the pressurized breathable gas and the atmospheric pressure.

US 5 320 093 A discloses a system for rapidly restoring ventilatory drive to an anesthetized patient after surgery. The end-expired carbon dioxide partial pressure is used as feedback to determine the amount of CO₂ to be added to the inspired breathing mixture of the patient's breathing circuit. The CO₂ added to the patient's breathing circuit increases CO₂ within the blood stream to a level sufficient to stimulate the central ventilatory center in the brain, thereby rapidly restoring ventilatory drive to the patient.

US 2004/003813 A1 discloses a ventilation system allowing setting of the conventional ventilation modes (i) assist control ventilation (ACMV); (ii) sychronized intermittent mandatory ventilation (SIMV); (iii) continuous positive airway pressure (CPAP); (iv) pressure-control ventilation (PCV); (v) pressure support ventilation (PSV); (vi) proportional assist ventilation (PAV); and (vii) volume assured pressure support (VAPS). four setting these modes, it is disclosed to set various ventilation parameters such as (i) a minute ventilation (Ve) level; (ii) a ventilator breathing frequency (f) level; (iii) a tidal volume (VT) level; (iv) a breathing gas flow rate (V) level; (v) a pressure limit level; (vi) a work of breathing (WOB) level; (vii) a pressure support ventilation (PSV) level; (viii) a positive end expiratory pressure (PEEP) level; (ix) a continuous positive airway pressure (CPAP) level; and (x) a fractional inhaled oxygen concentration (FIO2) level.

### SUMMARY OF THE INVENTION

The invention is defined by the appended claims. The embodiments disclosed herein are considered as examples, unless defined as embodiments of the invention. Many heretofore unmet needs are met and problems of the prior art are solved with the innovative ventilator embodiments according to the principles of the invention. Such features and capabilities may preferably or optionally include, among other elements and for purposes of illustration and example but not for purposes of limitation, improved and more accurate ventilation capabilities than has been possible with the many prior attempts.

The present invention provides a ventilator system for assisting in the respiratory function of a patient under the direction of a clinical as featured in the independent claim. Preferred embodiments of the present invention are featured in the dependent claims.

In one preferred configuration, a ventilator or ventilator system for assisting the respiratory function of a patient under the direction of a clinician contemplates the ventilator having a computerized, operation controller or control module or computing device that is in electronic communication with an intra-ventilator and/or extra-ventilator electrical or data circuit or data network. The controller also preferably includes a display, which can be a touch-screen display or any other suitable display, including an application-specific, customized display that incorporates data input or receiving devices into the display, with or without a touch-screen capability.

The controller or computing device also preferably includes a memory or storage capability that can include hard disk drives, removable drives and any desired form of storage device. Input devices are also desirable and can include keyboards, mouse pointers, data entry tablets, voice-activated input devices, and electronic media reading devices, among many others.

Additionally contemplated input devices include wired and wireless communications components for networking, data transfer, data capture, and data monitoring such as monitoring communications from electro-impedance tomography devices, ultrasound equipment, computed and computer-aided tomography devices, digital output fluoroscopes, x-ray equipment, magnetic resonance imaging and spectroscopy equipment, minimally invasive surgical and bronchoscopic visualization devices, and similarly capable equipment.

The ventilators also preferably include a gas supply pump and/or pressurized gas source. The gas pump or source supplies positive pressure gas to the patient through a gas circuit or network of pipes, tubes, hoses, or other types of conduits. The gas network or circuit may also include at least one and more preferably a plurality of valves and supply and exhaust ports. More preferably, a number of valves may be incorporated that can be electronically in communication with the controller or computing device for actuation.

Such valves can typically be included in line with the supply and exhaust ports and can be operable in cooperation with the various types of sensors discussed elsewhere herein. What are often referred to as back-check valves, which enable fluid flow in one direction but which prevent fluid flow in an opposite direction. Such check valves may be included in the gas circuit or network to protect various components and the patient from unexpected and/or undesirable pressures or pressure shock. In this way, protection can be afforded to the patient, pump or pressurized gas source, sensors, and other equipment.

Other preferably optional examples are directed to ventilation and control in negative pressure applications and the contemplated gas supply pump or pressurized gas source also may preferably incorporate, either alone or in combination with any of the other features described elsewhere herein, a negative pressure or vacuum capability that is contemplated to be compatible for use with negative pressure thoracic or full-body cylinders, which are also sometimes referred to by those skilled in the ventilation and respiratory technical fields as single, biphasic, or multiphasic iron-lung or cuirass ventilators.

More preferably, the ventilators also preferably include any number of optional detectors, sensors, or detection devices, that can be used alone or in any combination to sample and determine pressures of the supplied gas, inspired gas, expired gas, at rest, inflection point, and many different types of dynamic patient airway pressures. Other useful sensors can include peripheral, central, and airway gas concentration sensors that can be positioned extracorporeally in the case of well-known capnometers (for detecting carbon-dioxide concentrations) or infrared oximeters (for detecting oxygen concentrations).

These types of devices can be attached to extremities such as fingers, toes, or ear lobes, which make it very convenient to sample, monitor, and detect peripheral concentrations or saturations of carbon dioxide (SpCO₂) and oxygen (SpO₂). For other contemplated ventilation applications, central line catheters or other techniques can enable monitoring of arterial O₂ and CO₂ gas concentrations (PaO₂,PaCO₂), which can have clinical value in certain ventilation modes of operation or protocols discussed elsewhere herein. For purposes of the disclosure, such pressure and gas concentration sensors are more preferably configured to electronically communicate with the contemplated controller or computing device of the ventilator so that the real-time and/or near-real-time data can be monitored as described in more detail below.

The inventive ventilator also contemplates incorporation of any number of equally suitable fluid flow rate sensors that also may be adapted to communicate with the data network, the data circuits, and/or directly with the controller or control module or computing device, and either wirelessly or over wired connections. Such flow rate monitoring devices can be employed in multiple places in the gas network or circuit to monitor the total amount of gas supplied, inspired, expired, as well as the speed at which such activity is occurring or has occurred. With this information, pressure can be compared to total volume or rate of volumetric or mass flow of gas so that the novel ventilators can better control and ensure proper ventilation of the target respiratory system of the patient.

Additionally, such controls can improve the accuracy with which pressurized gas is supplied to the patient, and can lessen the risk of lung injury by monitoring and keeping gas flow rates within acceptable protocol limits. In any number of possible preferred configurations, the sensors can be arranged as a sensor array to simultaneously monitor any one or any number of ventilation-related parameters so as to maximize control over the ventilation procedure to ensure the best possible protocol implementation under the circumstances. In many preferably optional configurations, any of the noted sensors may be positioned proximate to and/or about the gas supply and/or exhaust ports, among other places in the gas network or circuit.

A command module, command routine, algorithm, commander, firmware, or program may preferably be resident in the memory or storage components of the controller or computing device. The command module is preferably operative to control the sensors, to control the supply pump, to receive communications or images or information from other devices, to receive input from the clinician or another via any of the contemplated input devices, and to operate the display to present prompts and/or display important information pertaining to the ventilation process. The command module may also be optionally configured to preferably communicate various ventilator information to other devices, to other wireless or wired networks, to the display for contemporaneous viewing, and to other remote devices and locations as may be desired.

Preferably, the control module may adjustably and/or variably actuate the pump or pressure source to vary the volume and/or pressure supplied thereby. Further, the control module or command routine may more preferably be modified to also automatically, manually, or otherwise operate any of the plurality of valves, either alone or in combination with the control of the pump, for even more rigorous control over the pressure, volume, flow rate, and gas supply cycle times available for use in ventilating the patient. More preferably, the control module or command routine may preferably be adapted to coordinate such control of the valves with sampling of or receipt of information from any of the contemplated sensors to establish increased accuracy in sampling one or more pressure readings, gas concentrations, and/or volume or mass flow rates anywhere in the gas circuit or network, or the patient undergoing ventilation.

The present disclosure also contemplates operational compatibility with any number of conventionally accepted, investigational, and experimental ventilation modes. More preferably, the operational capability enables many heretofore unavailable hybrid modes wherein the innovative ventilator automatically changes its mode of operation in response to patient progress or difficulties. For example, the ventilator can be configured to commence ventilation in a mandatory breath mode, and to monitor various patient pressure, volumetric, and gas concentration responses, among other responses, that may indicate a patient who was formerly heavily sedated and unable to breathe, has suddenly started to attempt to breathe spontaneously.

Upon such detection, the inventive ventilation will automatically switch among many modes of operation including from full-support mandatory breath modes, to various modes having lesser degrees of breathing support, so as to cooperate with the patient's attempts to breathe independently. Additionally, if the patient relapses and discontinues spontaneous breathing attempts, the ventilator will revert to full, mandatory breath mode. To enable such capabilities, the new ventilator is preferably preconfigured with various automated and reconfigurable modes of operation. For purposes of assisting clinicians with selecting fully automated modes of operation, or to enable partial or fully customizable modes of operation, the optionally preferred configurations enable the clinician to select any particularly desired automated mode of operation.

Additionally, the clinician may also select an automated mode of operation and then modify only the desired parameters. Even further, the clinician may ignore the fully automated modes, and may enter preferred settings to select a fully customized mode of operation suitable for purposes of any conceivable ventilation protocol or mode of operation. In one particularly useful configuration, the ventilator incorporates the controller or control module or computing device to have three primary operational modules, including, for purposes of example but not for purposes of limitation, an initial setup module, an adjustment and maintenance module, and a weaning module.

In turn, the initial setup module includes among other elements, an optimal end expiratory lung volume (OEELV) assessment mode that monitors a number of key patient parameters to ascertain and periodically compute the OEELV. The adjustment and maintenance module includes oxygenation, recruitment, and ventilation modes of operation and protocols that are tightly constrained to rigorously and aggressively monitor and protect the key aspects of these ventilation operational modes. This is accomplished using precisely bounded monitoring paradigms that enable very gradual and extremely accurate changes to manage CO₂ ventilation, to ensure optimal oxygen saturation, and, when needed, to exercise and maximize alveolar recruitment and prevent derecruitment. If any parameters experience unexpected or uncontrolled hysteresis, alarm events are triggered to enable intervention.

The weaning module includes an initial weaning protocol that enables close monitoring and small, slow changes to assess patient response to reduced ventilator support with rapid fall back to full support as needed. With positive patient response, the initial weaning protocol enables complete ventilator reconfiguration into subsequently less supportive ventilation protocols for further weaning. Also included in the weaning module is an airway pressure release ventilation or APRV protocol mode wherein spontaneous patient breaths are closely monitored so that support can be weaned as the patient gains control and consistency. With continued improvement, control is passed to the continuous positive airway pressure (CPAP) protocol mode, which cycles up to a maximum CPAP support mode that is then gradually reduced to a minimal support mode until an extubation pressure is reached, where after the patient is completely weaned from the ventilator.

For purposes of achieving these various modes of use, any of preferred or optional variations of the inventive ventilator may be predefined with or may receive and capture a number of parameters that can control how the ventilator operates in its various modes of operation. The controller or computing device may access stored parameters, may obtain new parameters from remote devices via wired or wireless communications, and may accept user input via the noted touch-screen display or any of the other input devices. Whatever the source of the operational settings or parameter, the information is typically stored in a database or an array that is stored in the memory or storage of the controller or computing device. In one optionally preferred embodiment, these parameters are accessibly stored in one or more initialization parameter database(s), which may be resident in the controller memory or storage.

These initialization parameters can be accessed and displayed or communicated to any other device. Alone or in combination with this database, additional subsets of parameters may be grouped together in arrays such as one or more model patient data arrays or elements, which can be predefined to represent optimum ventilator settings that are well suited for a particular type of presenting patient or disease.

For purposes of illustration, but not for purposes of limitation, such data arrays or initialization parameter databases may include, among other parameters and information, a positive end expiratory pressure (PEEP), a target peripheral O₂ concentration or an SpO₂ quantity, an end tidal CO₂ or etCO₂ quantity, a fraction of inspired O₂ or FiO₂ quantity, a high pressure or P(high) that defines the maximum inspired pressure during mandatory or positive pressure assisted breaths, a low pressure or P(low) that can define a minimum pressure to be used during expiration and which can be zero or non-zero. Other parameters can include a high time or T(high) that represents a period of inspiration and a low time or T(low) that can represent a small period of time during which expiratory gas is expelled.

It may also be optionally preferred to include predefined or predetermined pressure change increments or pressure increments, P(inc), and time increments, T(inc), which can be any amount, and for which there can be multiple different preset increments that can be used as needed and so that clinical intervention may not be needed in more automated modes of operation. It has also been found to be sometimes desirable to store one or more tidal volumes, respiratory frequencies for mandatory breaths, to establish and store one or more pressure-volume curve slopes, and to establish one or more trigger pressures that enable the ventilator to detect a pressure drop trigger, which may indicate the patient is trying to spontaneously breathe.

In yet further optionally preferred variations, the ventilator may also be configured so that the command module can receive such initialization settings from the user or the clinician via the input device. Such settings can include those described elsewhere herein or any other possibly desirable parameters that can improve the use of the ventilator. Once any preferred settings and/or parameters are entered into the controller and/or command routine, the supply pump is actuated to begin ventilation within the constraints of the selected automated program or settings or the manually entered parameters and settings.

During operation, the command module or routine samples, polls, or otherwise communicates with, any or all of the sensors in the array and measures the patient's actual data. A series of such sensor readings may be sampled so that an entire array of such data elements can be used to monitor patient response and ventilator performance, and to adapt the ventilator performance in response to patient status and condition. For purposes of example without limitation, sensor data that can be gathered may optionally include a patient SpO₂ partial pressure (PP) or quantity, a patient etCO₂ PP or quantity, a peak expiratory flow rate (PEFR), an end expiratory lung volume (EELV) and a spontaneous frequency or machine respiratory frequency.

Once measured or sampled, the actual patient data array elements can be compared by the command module to any of the stored data to ascertain ventilator performance and patient response. For further example, such actual data may be compared to the one model patient data array. In this way, it can be determined whether the patient is responding favorably to ventilation. In another example, if the patient is responding well to ventilation, then a comparison between the patient's SpO₂ and etCO₂ and a comparable model patient data set would be acceptable. If acceptable, then the control routine can compute or generate a flag or Boolean value such as an SpO₂ goal value and an etCO₂ goal value that can be set to true, meaning the actual patient measurements indicate all is well. If not, then a false flag can be generated to enable the control routine to modify its behavior or seek clinical intervention by generating an alarm condition. Additionally, the control routine can poll pressure sensors and flow sensors during certain points in the inspiration and expiration phases of ventilation to ascertain an optimal end expiratory lung volume (OEELV), which can provide clinically relevant feedback identifying patient response and ventilator performance.

In yet other optionally preferred configurations, the ventilator may be configured to modify its behavior in response to unfavorable patient response. For further example, assume the SpO₂ was unfavorable and the SpO₂ goal value is false, which indicates undesirable oxygenation. As discussed in more detail elsewhere herein, the command module can preferably determine that increased or modified adjustments in ventilation are warranted to achieve the desired SpO₂ level. To that end, the command module will adjust the operation of the pump, and functioning of the valves, and perhaps the concentration of supplied oxygen in the pressurized gas supply, and may thereby increase the P(high) the pressure increment, it may increase the T(high) by the time increment. In the alternative, it may be instead preferable to only modify the various operational parameters of the ventilator to increase or decrease the FiO₂ quantity.

More preferably, in circumstances where the patient is responding well and it is warranted to start gently weaning from the ventilator support, the command module can instead set a flag or Boolean constant, such as an initial weaning value to be true, which can serve to notify other modules of the ventilator that weaning may begin. Conversely, if the patient is experiencing difficulties that may include non-perfused pulmonary dead space, it may be advantageous to modify the ventilator behavior to encourage recruitment of alveolar tissues. In certain circumstances, it may be advisable to generate an alarm signal seeking intervention. In other less challenging circumstances, it may desirable to set a recruitment flag or Boolean value to notify other control routine modules that a recruitment process is advisable. In this circumstance, the control routine can modify the pump and valve operation to establish operation suitable for recruitment, which can include increasing P(high) by one or more P(inc), increasing T(high) by one or more T(inc)s, and/or adjusting T(low) by one or more T(inc)s.

In other situations where recruitment may not be indicated, it may be desirable to increase lung oxygenation. If so, the controller or computing device may make adjustments to the ventilator operation whereby an oxygenation flag or Boolean value is set to be true, which can invoke an oxygenation module that can poll the sensors to measure the peak expiratory flow rate and then compute an angle of deceleration of gas flow so that an appropriate time adjustment may be made, or so that T(low) may be decreased by one or more T(inc).

In yet other equally useful modes of operation, the ventilator can invoke an alveolar ventilation approach wherein the command module compares the spontaneous frequency to the machine respiratory frequency, ascertains the P(high), T(high), computes a minute ventilation (MV) value, adjusts the supply pump and valves to increase T(high) and P(high) by respective T(inc) and P(inc). Similarly, a minute ventilation and recruitment module may be invoked wherein the MV is computed as a function of the currently in use P(high) and T(high), and adjustments are made to the P(high) and T(high).

In more favorable patient response scenarios, an initial weaning module may be utilized wherein the command module or command routine samples the machine respiratory frequency and spontaneous respiratory frequency to ascertain that spontaneous breathing is occurring at a certain rate. Comparing this rate to a predefined rate gives a good indication of whether an initial weaning protocol can be employed. If so, then the command routine can test for apnea and tachypnea. If neither condition is indicated, then the ventilator can be switched to a more suitable mode, such as an APRV mode, which makes it much easier for the intubated patient to breathe spontaneously.

As the patient who is experiencing initial ventilator weaning continues to improve, another mode of the ventilator enables a weaning protocol wherein spontaneous breathing and blood gas levels continue to be monitored while the P(high) is decreased while the T(high) is increased. In this way, the patient is encouraged to continue spontaneous breaths.

As improvements mount, further weaning is warranted. In this instance, the ventilator operates in another mode wherein weaning failure criteria can be considered in comparison to the actual patient data that is being monitored. In one suitable set of predetermined weaning failure criteria, an FiO₂ threshold, an SpO₂ threshold, a spontaneous tidal volume, a minute ventilation quantity, and an airway occlusion pressure (P0.1) are compared to the patient's actual values. If the patient fails to meet these criteria, then weaning is discontinued temporarily and more breathing support is given to the patient. In weaning failure, the control routine increases the P(high) and decreases T(high). Conversely, if the weaning failure criteria are passed by the patient's actual values, then the command module repeatedly initiates the cyclic weaning protocol wherein P(high) is decreased. The airway occlusion pressure P0.1 is measured and trended over time by a P0.1 module and is used to assess the work of breathing during spontaneous breaths. In the preferred embodiments, this is used to assess the impact of weaning and the resultant work of breathing, as is explained in connection with other modules elsewhere herein,

Assuming for further purposes of illustration that the patient continues improving, then the command module changes ventilator operation again, and monitors P(high) until a CPAP threshold is reached, which enables another conversion of the ventilator operation into a CPAP mode. This mode is much more comfortable for patients, and reduces the dependence of the ventilator. As the further improvements are manifested, the command module begins to gradually reduce the CPAP pressure until an extubation threshold pressure is reached. Also, it may be optionally preferable during the CPAP and other modes of operation to enable the controller to incorporate an automatic tube compensation pressure or ATC pressure which boosts the ventilator support just enough to overcome the frictional losses encountered when breathing through the gas network or circuit of tubing involved in use of the ventilator.

Using any of the physical configurations of the ventilators described elsewhere herein, one method of use involves entering settings via the input device including at least one of (i) an automated initialization setting and (ii) a parameter to be stored in the memory that includes at least one of (a) a PEEP quantity, (b) an SpO₂ quantity, (c) an etCO₂ quantity, (d) an FiO₂ quantity, (e) a high pressure, (f) a low pressure, (g) a high time, (h) a low time, (i) a pressure increment, (j) a time increment, (k) a tidal volume, (l) a respiratory frequency, (m) a pressure-volume slope, (n) a trigger pressure, and (o) a predetermined weaning failure criteria including at least one of an FiO₂ threshold, an SpO₂ threshold, a spontaneous tidal volume, a minute ventilation quantity, and an airway occlusion pressure (P0.1).

Next, the command routine receives the settings from the clinician via the input device and commands the controller to actuate the supply pump. This commences respiratory assistance to the patient whereby the gas circuit communicates with the patient using one each of the FiO₂ quantity, the high and low pressure, and the high and low time. Patient actual data array elements are measured by using the command routine to communicate with the plurality of sensors. Measurement of at least one of (i) a patient SpO₂ quantity, (ii) a patient etCO₂ quantity, (iii) a peak expiratory flow rate, (iv) an end expiratory lung volume and (v) a spontaneous frequency, is taken.

The command routine compares the patient actual data array to at least one of the settings and computes at least one of an SpO₂ goal value, an etCO₂ goal value, and an optimal end expiratory lung volume, which values are used to determine whether the patient should be initially weaned, undergo recruitment and increased oxygenation, or be maintained in an unaltered state of ventilation.

If the patient is improving, then measuring and comparing at least one of the patient actual data array elements to the predetermined weaning failure criteria to set a flag or Boolean weaning failure value to false is warranted (meaning the patient did not fail the weaning test). If passed, the cyclic weaning is initiated by adjusting at least one of the supply pump and the plurality of valves to decrease the P(high) by one or more pressure increments.

Also, to decrease the ventilator support even further, the T(high) is gradually increased and the P(high) is gradually decreased until the CPAP threshold is reached, where after the ventilator is switched into CPAP mode where it remains until the CPAP pressure may be decreased until the extubation threshold pressure is reached, after which the patient may be removed from the ventilator.

In other optionally preferred novel embodiments, any of the monitoring devices, sensors, computers, or computing devices may be connected with any of the other components wirelessly or with a wire. Any of the contemplated components may also be in communication with any of the other components across a network, through a phone line, a power line, conductor, or cable, and/or over the internet. In other alternatively preferred configurations, the resident software program may have numerous features that, for purposes of example without limitation, can be enabled.

More preferably, such resident software program and/or programs may enable any of the contemplated information to be communicated by text, voice, fax, and/or email messages either periodically, when certain predefined or predetermined conditions occur, such as predefined alarm events or conditions, and/or when anomalous, unexpected, or expected power readings occur and/or are detected.

These variations, modifications, and alterations of the various preferred and optional embodiments may be used either alone or in combination with one another and with the features and elements already known in the prior art and also herein contemplated and described, which can be better understood by those with relevant skills in the art by reference to the following detailed description of the preferred embodiments and the accompanying figures and drawings.

### BRIEF DESCRIPTION OF THE DRAWING(S)

Without limiting the scope of the present invention as claimed below, and referring now to the drawings and figures, wherein like reference numerals across the drawings, figures, and views refer to identical, corresponding, or equivalent elements, methods, components, features, and systems:
FIG. 1 shows a ventilator and system in accordance with the present invention;
FIG. 2 shows a schematic diagram of the operation of the ventilator and system of FIG. 1;
FIGs. 3a and 3b are schematic diagrams of an OEELV mode of operation of the ventilator and system of FIG. 1;
FIG. 4 shows a schematic diagram of the interrelationships between the modules of operation of the ventilator and system of FIG. 1;
FIG. 5 shows a schematic diagram of an oxygenation mode of operation of the ventilator and system of FIG. 1;
FIG. 6 is a schematic diagram of a recruitment mode of operation of the ventilator and system of FIG. 1;
FIG. 7 is a schematic diagram of a ventilation mode of operation of the ventilator and system of FIG. 1;
FIG. 8 is schematic diagram of an initial weaning mode of operation of the ventilator and system of FIG. 1;
FIG. 9 is schematic diagram of an ARPV weaning mode of operation of the ventilator and system of FIG. 1;
FIG. 10 is a schematic diagram of a CPAP weaning mode of operation of the ventilator and system of FIG. 1;
FIG. 11 is an area diagram of an OEELV mode and assessment of operation of the ventilator and system of FIG. 1;
FIG. 12 is an area diagram of an OEILV mode and assessment of operation of the ventilator and system of FIG. 1;
FIG. 13 is an area diagram of a spontaneous mode and assessment of operation of the ventilator and system of FIG. 1;
FIG. 14 is a schematic airway pressure versus time tracing for airway pressure release ventilation;
FIG. 15 is an airway pressure versus time tracing during the inspiratory P(high) phase of ventilation;
FIG. 16 is an airway volume versus pressure curve illustrating a shift from the inspiratory limb to the expiratory limb thereof;
FIG. 17 is an inspiratory and expiratory gas flow versus time tracing for airway pressure release ventilation;
FIG. 18 is an expiratory gas flow versus time tracing;
FIG. 19 is a set of expiratory gas flow versus time tracing illustrating determination of whether flow pattern is normal, restrictive or obstructive based on the shape of the tracing; and
FIG. 20 is a set of airway pressure versus time tracings illustrating ventilation weaning by successive reductions in pressure P(high) and substantially contemporaneous increases in time T(high).

### DETAILED DESCRIPTION OF THE INVENTION

Referring now to the various figures and illustrations, those skilled in the relevant arts should appreciate that each of the preferred, optional, modified, and examples of the ventilator and ventilator system 10 and method of operation contemplate interchangeability with all of the various features, components, modifications, and variations within the scope of knowledge of those skilled in the relevant fields of technology and illustrated throughout the written description, claims, and pictorial illustrations herein.

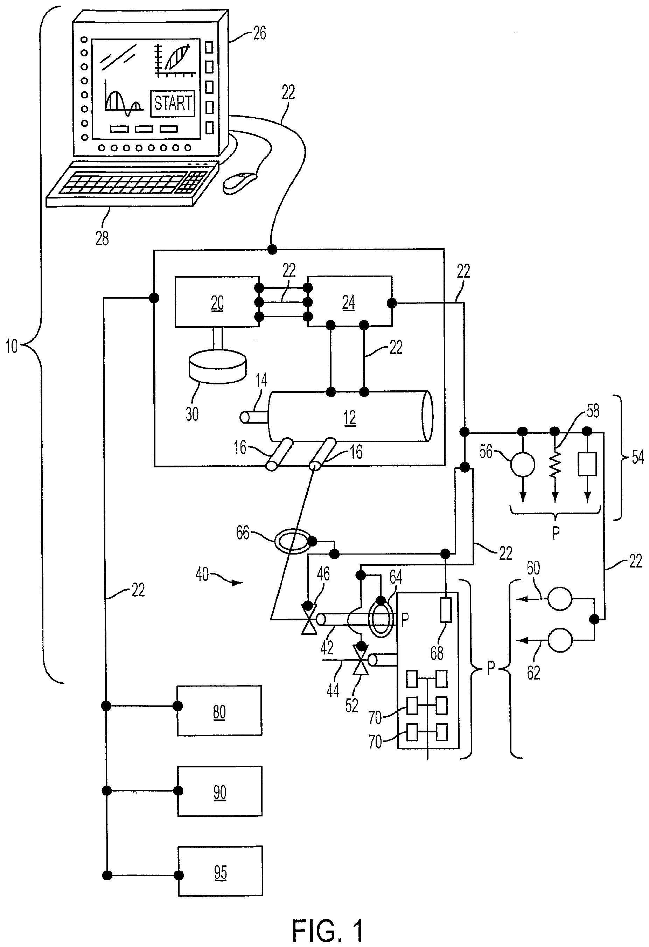

With this guiding concept in mind, and with reference now to FIG. 1, one possible embodiment of a ventilator and ventilator system 10 is illustrated, which is in communication with the patient P undergoing ventilation therapy. The ventilator and ventilator system 10 also preferably includes a gas supply pump and/or pressurized gas source 12 having a positive pressure port 14, and optionally a negative pressure port 16. The gas pump or source 12 supplies positive pressure gas 14 and can also supply negative pressure or a vacuum 16 for non-invasive negative pressure applications such as iron-lung or similar therapies. A wide variety of commercially available ventilators may be modified according to the principles of the invention and one such device includes what is referred as the model EvitaXL, which is available from Draeger Medical, Inc. of Telford, Pennsylvania, USA, and Lubeck, Germany.

The ventilator 10 also preferably includes a controller or control module or computing device 20 that is in electronic communication with an intra-ventilator and/or extra-ventilator electrical or data circuit or data network 22. The controller 20 also preferably includes a display 26. The display 26 may be a conventional device that receives unidirectional signals from the controller 20, but may also be any of a number of possibly preferred bidirectional devices such as a touch-screen display that can be used as an input device 28, and which may also have a data entry capability such as a built-in keyboard or keypad similar to the keyboard input device 28 shown in FIG. 1.

The controller or computing device 20 also preferably includes a memory or storage capability 24 that can include flash drives, optical media, hard disk drives, solid state disk drives, random access memory, non-volatile memory, removable storage devices, remote internet-based storage devices, network appliance-type devices, and the like.

Typically, the gas supply pump or pressurized gas source 12 communicates positive or negative pressure to the patient P through a gas circuit or network of tubes 40. The gas network or circuit 40 may also include an inspiration or supply port 42 and an expiratory or exhaust port 44. A number of valves are usually also included to control and meter fluid flow and would preferably include a supply valve 46, a sensor valve (not shown), and an exhaust valve 52, all of which would likely be in communication with the controller 20 via the data network 22 so that the command module 30 may control and operate the valves automatically to start and stop ventilation and to control pressure and flows rates to the patient P during operation.

The supply and exhaust valves 46 and 52 may be also operable to periodically close for short periods of time to enable pressure sensors to obtain various static pressure readings. Additionally, the sensor valve may be operable to close such as to protect various sensors from pressure circuit transients and to prevent spurious readings such as when the ventilator 10 may be automatically responding to patient P improvements or relapses by changing modes of operation from mandatory breathing support to augmentative support modes.

In addition to the contemplated input devices described elsewhere herein, there may be certain diagnostic imaging devices that can be incorporated into the operation of the ventilator 10 to communicate quantitative pulmonary function information such lung volume, dead space ratios, and the like. Additional and possible useful devices may also include, for purposes of example without limitation, electro-impedance tomography devices 70, ultrasound equipment 80, computed and computer-aided tomography devices 90, and other types of Doppler imaging sensors 95 that may enable various quantitative or subjective pulmonary function imagery.

Various types of optionally preferred detectors, sensors, or detection devices, also have utility to enable precise control and analysis of volumetric and mass flow rates as well as pressures of the supplied gas, inspired gas, and expired gas, which in turn enables calculation of various other static and dynamic pulmonary function parameters, as is discussed in more detail elsewhere herein.

With continued reference to FIG. 1, a group of sensors 54 can be arrayed proximate to the ventilator 10 and patient P. An oximeter or O₂ saturation sensor 56 may be used peripherally to ascertain peripheral or venous O₂ content SpO₂ and a capnography sensor or capnometer or CO₂ sensor 58 may be used to determine end tidal or peripheral CO₂ saturation levels (etCO₂, SpCO₂). For certain applications involving long term supine ventilation, it may be desired to also monitor arterial blood gas concentrations, among other parameters. In these instances, invasive methods can be used such as central line catheters to assess pulmonary arterial O₂ and CO₂ levels using a PaO₂ sensor 60 and/or a PaCO₂ sensor 62.

It is also optionally preferred to monitor various airway pressures, volumetric, and mass flow rates so that patient P response can be continuously assessed. For purposes of monitoring airway pressures, an airway pressure sensor or pressure gauge 64 can be placed in a number of places along the gas network or circuit, and is more preferably positioned proximate to the supply and exhaust ports 42, 44 at the intubation site of the patient P. An airway flow sensor 66 can be similarly positioned to enable monitoring of volumetric flow rates of inspiratory and expiratory gases. In certain applications, it has been found desirable to employ thoracically mounted strain gauges to enable monitoring of chest movement during pulmonary breathing cycles, which can be an additional source of volumetric pulmonary patient P function as well as a source of patient P work expended for spontaneous breaths.

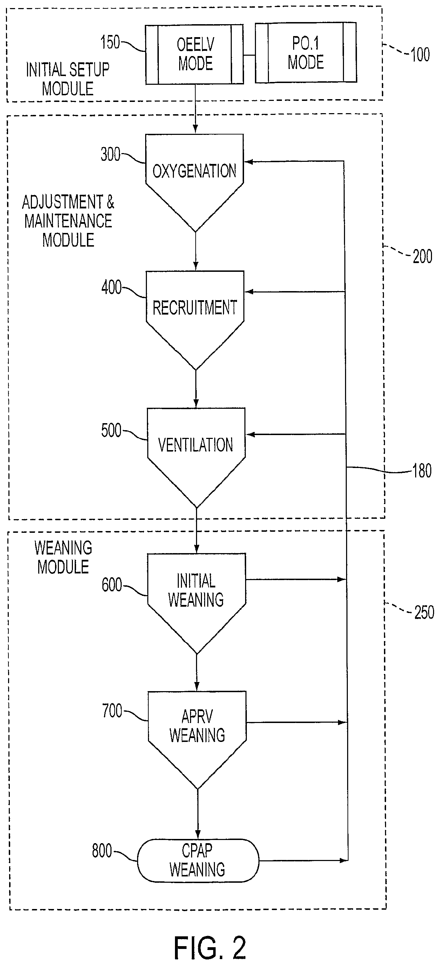

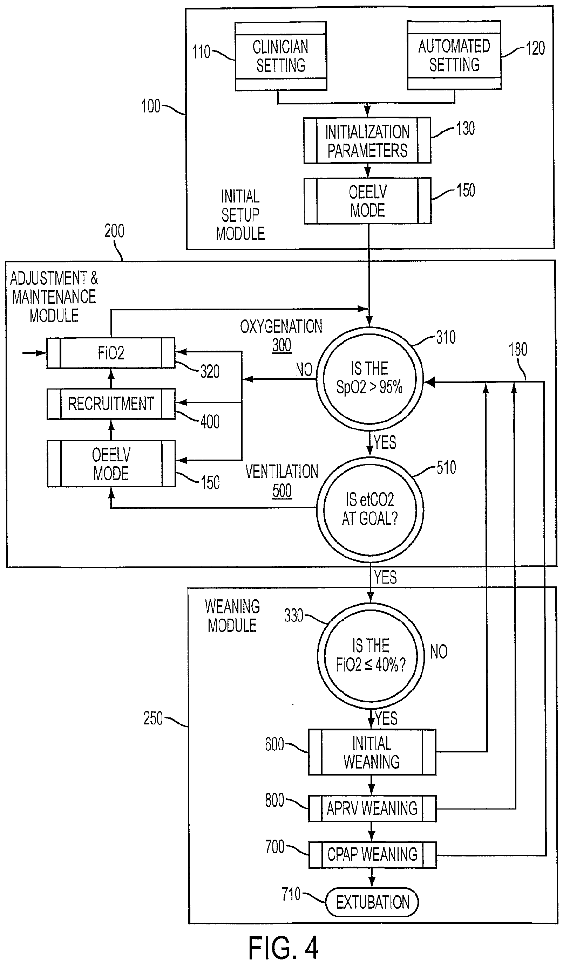

With reference now also to FIG. 2, the ventilator 10 also incorporates the control module 20 and/or the command module 30 to include three primary operational modules, including, for purposes of example but not for purposes of limitation, an initial setup module or protocol 100, an adjustment and maintenance module 200, and a weaning module 250. During initial ventilator 10 startup, a number of initial parameters are set based upon input from the clinician or by accessing a predefined set of parameters. With reference now also to FIGS. 2, 3, and 4, the preliminary initialization routines will be described. In FIG. 4, it can be seen that the clinician may enter their preferred settings 110 into the display 26 or input device 28. In the alternative, any number of possible predefined automated settings 120 may be accessed and used as defined or customized in whole or in part to prepare the ventilator 10 for operation. Once the clinician or automated settings 110, 120 are selected, the settings 110, 120 are populated with various other initialization parameters 130 during the operation of the initial setup module 100. As the operation of the ventilator 10 commences, the command module 30 invokes an OEELV mode or assessment routine 150.

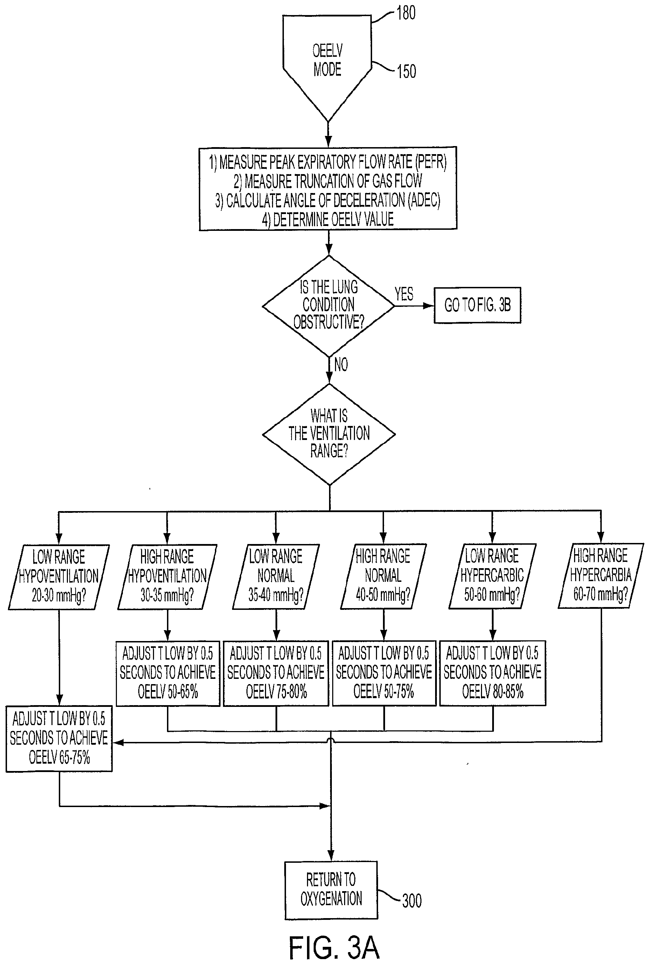

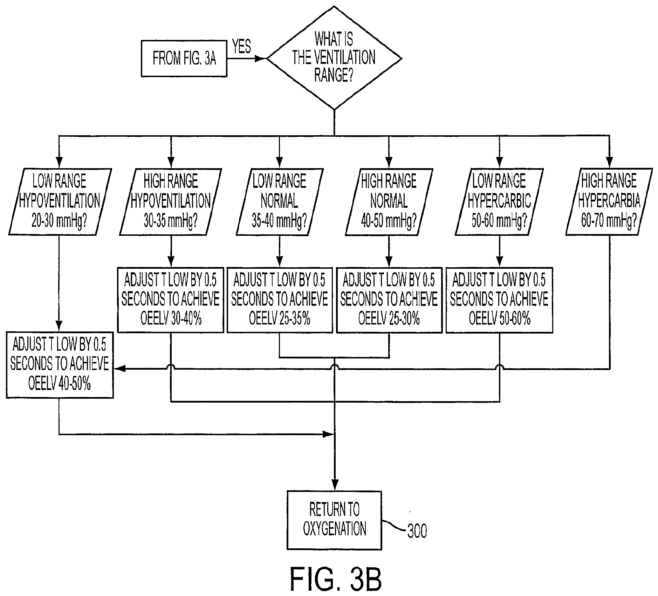

With specific reference now to FIGS. 3a and 3b, it can be seen that the initial setup module 100 includes the optimal end expiratory lung volume or OEELV mode or assessment routine 150. The OEELV mode 150 periodically and on demand will determine a ventilation range as a function of obstructiveness of the lung and the hypoventilation, normal, and/or hypercarbic condition of the patient P. As part of the evaluation, the OEELV mode 150 determines whether the computationally ascertained OEELV is in the range appropriate for the conditional status of the patient P. For example without limitation, if the patient P has obstructive lungs, and is experiencing high range hypoventilation, then an appropriate or desired OEELV should be in the range of about 30% to 40%.

If the computationally ascertained OEELV is higher than this range, then the T(low) parameter is increased by 0.05 seconds. Conversely, if the computationally ascertained OEELV is lower than the desirable range of 30% to 40%, then the T(low) parameter is lowered with the intent to achieve the appropriate OEELV range. In this way, the novel OEELV mode or assessment protocol 150 can execute very fine adjustments of actual OEELV to stimulate optimum conditioning of the ventilated patient's P pulmonary response. To wit, adjustments of 0.05 seconds in T(low) will enable slow and gradual optimization of the OEELV best suited to the disease modality. Although for purposes of illustration and explication of various aspects of the disclosure, increments of 0.05 seconds or other amounts of time have been described. However, the principles of the disclosure in this aspect are also suitable for even more gradual changes in time, and can include milliseconds and smaller and larger orders of magnitude.

Once T(low) is set, the OEELV mode 150 relinquishes control for a period of time and the command module 30 again resumes control to then invoke the adjustment and maintenance module 200, which includes an oxygenation mode 300, a recruitment mode 400, and a ventilation mode 500. The module 200 and its component modes 300, 400, and 500 include protocols configured to rigorously monitor and protect the key aspects of the patient's P physiological ventilation and pulmonary response profile to enable maximized recovery and weaning with minimum pulmonary injury risk. During the ensuing ventilation process, the patient's P SpO₂ and etCO₂ are continuously monitored via the respective SpO₂ and etCO₂ sensors 56, 58 to ensure a target or goal of SpO₂ of at least about 95% and etCO₂ of no more than between about 34 to 45 mm Hg are maintained (FIG. 4).

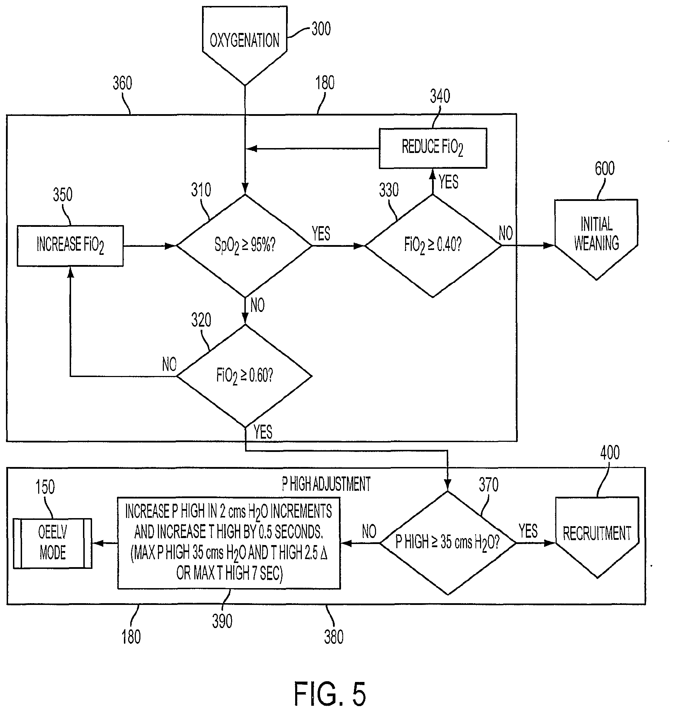

The command module 30 next passes control to oxygenation mode 300, which is described in more detail specifically in FIGS. 4 and 5. As the oxygenation mode 300 assumes control for a short period of time, the SpO₂ is again assessed so that adjustments may be made as required in the fractionally inspired O₂, which is otherwise referred to as the FiO₂ parameter 360. Once adjustments are made to FiO₂ 320, 340, 350, the command module 30 cooperates with the oxygenation mode 300 to assess whether a P(high) pressure adjustment must be made or whether the initial weaning mode 600 is invoked. If the patient P is responding well, and if the FiO₂ 330 and SpO₂ 310 quantities are suitable, then control will be transferred to the initial weaning mode 600, which is discussed in more detail elsewhere herein.

In the alternative, the oxygenation mode 300 and command module 30 assess the P(high) condition 380. If P(high) is adjusted 390, then another iteration of the OEELV mode 150 is also conducted to support the optimum OEELV mode 150 discussed earlier. As control returns to the oxygenation mode 300, P(high) is again assessed 370 to determine whether recruitment mode 400 is warranted or whether P(high) must again be adjusted. Assuming for purposes of further illustration that recruitment mode 400 is indicated, the oxygenation mode 300 relinquishes control to command module 30, which invokes the recruitment mode 400.

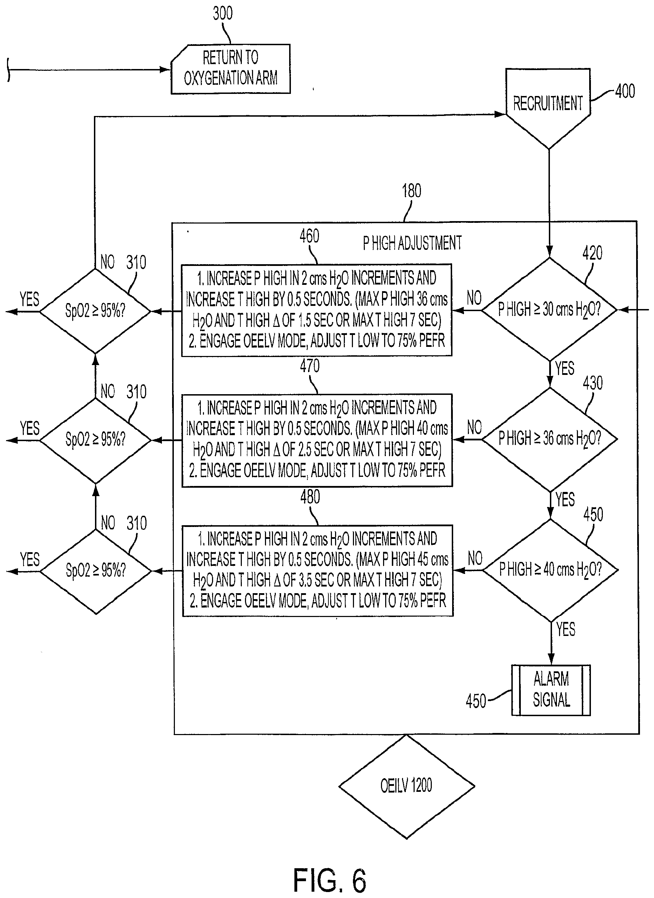

Referring now also to FIG. 6, recruitment mode 400 reevaluates the P(high) condition in a different context 420, 430, 450, as depicted in more detail in FIG. 6. In the circumstance where P(high) becomes unmanageable 450, an alarm signal 410 is annunciated to affect immediate intervention. Otherwise, P(high) is adjusted 460, 390, 480 to improve the pulmonary condition of patient P and the SpO₂ is again iteratively re-examined 310 while recruitment mode 400 continues attempts to increase lung surface, reduce dead space, and re-inflate alveolar units as much as possible until SpO₂ values 310 indicate the need for oxygenation mode 300. Feedback of information from other concurrently running modes may be sampled periodically via feedback loop 180 by command module 30, which can interrupt recruitment as needed and transfer control or invoke a more important mode when required by patient P physiology. For example, if command module 30 detects inbound information from feedback loop 180 describing increased etCO₂ values approaching or exceeding desired limits, control can revoked by command module 30 so that ventilation mode 500 can be invoked.

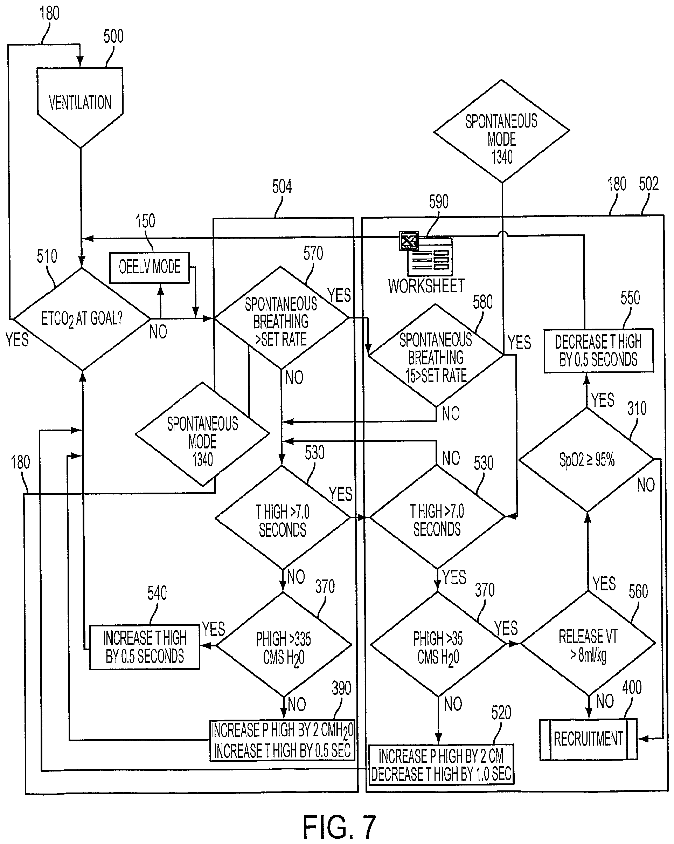

With continued reference to the previously discussed figures and now also to FIG. 7, command module 30 invokes ventilation mode 500 to redress an actual or approaching out of limit etCO₂ condition. The ventilation mode 500 re-evaluates end tidal CO₂ levels 510, reassesses OEELV conditions, and then assesses patient P breath spontaneity 570 against the set rate or respiratory frequency values obtained from the clinician 110 or automated settings 120. If breathing spontaneity remains at or below the set rate 570, then an alveolar ventilation sub-mode 504 is affected to adjust T(high) 530, 540 and P(high) 370, 390 as may be needed to further optimize pulmonary response.

However, as patient P recovers pulmonary responsiveness and breath spontaneity improves beyond the set rate of, for purposes example without limitation, to a rate of 15 spontaneous breaths over the set rate, then an alveolar ventilation sub-mode 504 is effected whereby T(high) 530, P(high) 370 are adjusted separately, and then in combination 520 for lower values of P(high). For higher values of P(high), the minute ventilation sub-mode 506 evaluates Vt 560 to determine whether recruitment mode 400 is warranted. If not, then SpO₂ is again verified 310, T(high) is adjusted 550, and the patient P is examined against a tachypnea assessment worksheet 590 that is a function of the set rate or respiratory frequency and the actual patient P rate defined in the assessment worksheet 590. As with other modes, the command module 30 continues to poll for information inbound on the feedback loop 180 so that control can be instantly seized to maintain optimal pulmonary response parameters across the suite of continuously monitored variables.

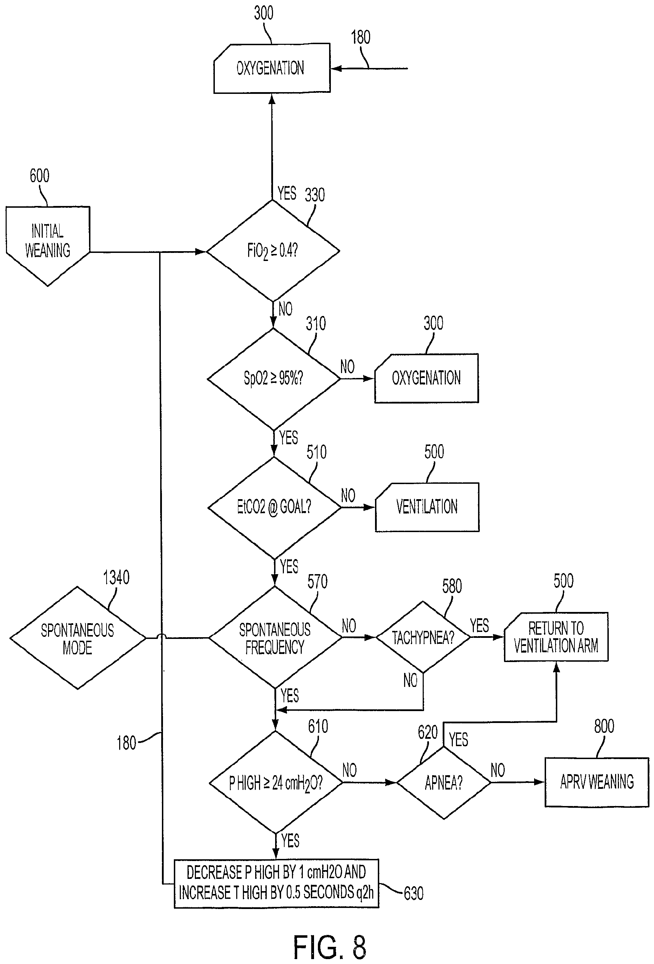

Attention is now invited also to FIG. 8 with the hypothetical suggestion that command module 30 recalled control from the ventilation mode 500 for another pass through the oxygenation mode 300, and the parameters were evaluated favorably for the command module 30 to invoke the initial weaning mode 600. As with other modes, the FiO₂ 330, SpO₂ 310, etCO₂ 510, breathing spontaneity 570, and possible tachypnea 580 are re-evaluated. Assuming patient P responds well, then P(high) is assessed 610 and if warranted, the spontaneity of breathing is compared against apnea parameters 620. The markedly improving patient P will then experience one of the very novel aspects of the inventive ventilator 10 as the command module 30 reconfigures the ventilator 10 away from the mandatory breath control mode and invokes an assisted breathing mode.

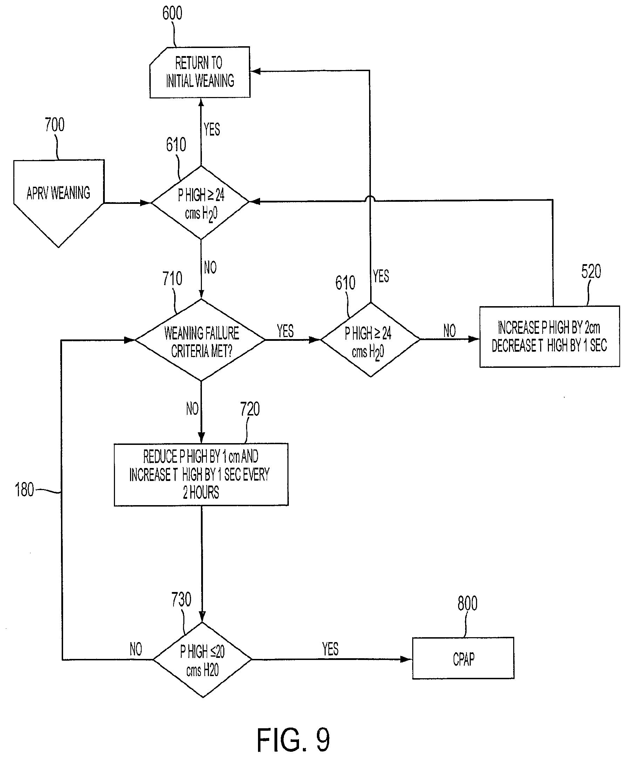

With reference now also to FIG. 9, those knowledgeable in the pertinent fields of expertise will appreciate that the command module 30 invokes an airway positive release ventilation or APRV mode 700, which can be much more comfortable for the recovering pulmonary patient P. Here too, the APRV mode 700 re-verifies the pulmonary conditioning of the patient P and examines P(high) 610. However, unlike other modes, the APRV mode 700 institutes a new parameter evaluation set referred to herein as the weaning failure criteria 710. These criteria evaluate the patient P against a more rigorous series of critical pulmonary physiological conditions to ensure the patient P can withstand the added stresses of substantially less gradual changes in the ventilation mode of operation. Before the command module 30 discontinues the mandatory breathing modes of operation, the patient P must pass these weaning criteria 710. If the patient P fails the weaning criteria 710, then the command module 30 re-invokes the initial weaning mode 600, or another mode if feedback loop 180 alerts the command module 30 to a more urgent requirement.

Assuming the weaning failure criteria 710 are met, however, then the patient P is deemed able to withstand greater changes in the pressure-volume slope profile being induced by the ventilator and ventilator system 10. Accordingly, the APRV mode 700 effects additional adjustments 720 to wean or reduce the patient's P reliance on the mandatory breathing modality of the ventilator 10. This weaning process and re-evaluation 720, 730, 710 continues to iterate if well tolerated by the patient P until P(high) is less than or equal to a pressure of only 20 cm H₂O. At this point, the patient P is recovering well and absent an important indication to the contrary over the feedback loop 180, the command module 30 again completely reconfigures the operational profiles of the ventilator 10.

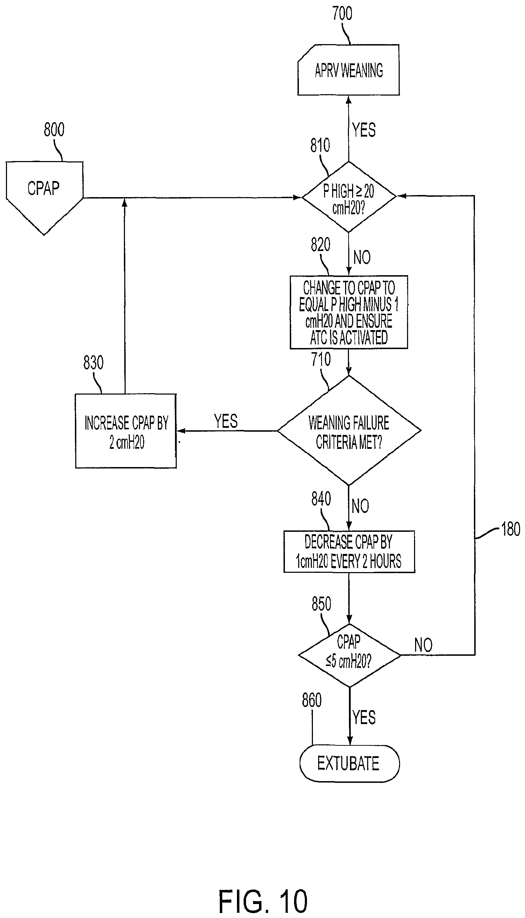

As illustrated in detail in FIG. 10, the commander or command module 30 invokes the continuous positive airway pressure (CPAP) mode 800 in a maximum CPAP assistance mode, which speeds up the process of removing the patient P from reliance on the ventilator 10. However, the patient P continues to be evaluated against the weaning failure criteria 710, and for gross and undesirable deviations from acceptable pulmonary response limits As the patient's P recovery accelerates, the CPAP mode 800 decreases assistance 820, 840, 850, until an extubation pressure 860 is reached. Hereafter, the clinician intervenes and extubates the weaned patient P.

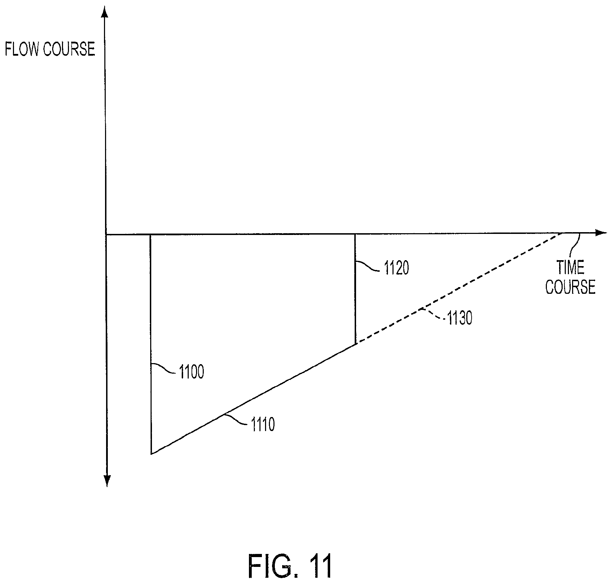

Among many possible modifications to any of the embodiments of the inventive ventilator 10, one particularly useful variant includes a modified OEELV (not shown), that can be incorporated as an improvement to the OEELV mode 150, or which may be included as an independent mode capable of operating and cooperating with OEELV mode 150. With continued reference to the various figures and especially to FIGS. 3a and 3b, and with reference now also now to FIG. 11, those having an understanding of the relevant areas of technology may recall that optimal end-expiratory lung volume (OEELV) is derived by using the elements and reference points information acquired during the P(low)/T(low) cycle as is described elsewhere herein. The proposed and optionally preferred OEELV model 50 is ideally functioning for the duration of the ventilation therapy and is operative to continuously optimize EELV, or end expiratory lung volume, of the therapeutic patient P.

The derived OEELV is a function of disease state (see, e.g., FIGS. 3a and 3b), and the patient's P pulmonary responsiveness to the oxygenation mode 300, recruitment mode 400, and ventilation mode 500. In a compliant patient P, the OEELV mode 150 optimizes EELV by adjusting the T(low) time period. Preferably, the OEELV mode sub-module 150 also validates the acquired information by using multiple sampling, averaging, and various statistical methods overtime for validation and error detection.

Adjustments of the OEELV are based upon the elements and flow and time reference points acquired during the P(low)/T(low) cycle. Flow and time reference points within the flow/time area, which is established by the P(low)/T(low) cycle, may be used to measure and calculate changes occurring in lung volume during the P(low)/T(low) cycle. For purposes of example and further illustration, but not for purposes of limitation, and looking again to FIG. 11, the preferred OEELV mode 150 measures the peak expiratory flow rate (PEFR) 1100, the decay phase 1110, and the truncation phase 1120 to calculate the angle of deceleration (ADEC) of gas flow and the termination of the flow of gas to determine optimal T(low) adjustment. The OEELV mode 150 thereby enables a heretofore unavailable dynamic adjustment, which more accurately and more responsively establishes and maintains the most optimal actual OEELV of the patient P.

An analysis of FIG. 11 ought to reveal to those skilled in the arts that an extrapolation phase 1130 may be used to calculate a residual volume and pressure as a function of time. The PEFR 1100 of FIG. 11 represents the rapid depressurization evidenced by the relaxation and recoil of the thorax after the machine breath. The decay phase 1110 represents the decaying energy drive and the downstream resistance to gas flow. The flow termination phase or truncation phase 1120 establishes the location or region where the flow can be determined either as a function of the disease process, or the parameter setting that was input by the user or clinician. The extrapolation phase 1130 can be used graphically and/or algebraically to determine and calculate pressure, volume, and time.

As described elsewhere herein, and with continued attention to FIG. 11, we recall that the OEELV sub-module 150 as well as the OEELV mode 150 both measure the peak expiratory flow and the truncation of gas flow, and then uses this information to calculate the ADEC or angle of deceleration, which is in turn used to establish the ideal OEELV value. With this approach, it should be observed that changes in the truncation point will change the angle of deceleration. When the resulting angle becomes less acute, the resultant observation is that recruitment has occurred. Conversely, when the angle becomes more acute, derecruitment is indicated.

Therefore, the OEELV mode 150 suggest adjustments to at least one of P(high), P(low), T(high), or T(low). In the instance where derecruitment is detected, P(high) or T(high) should be increased, or T(low) should be decreased, or some combination thereof should be effected. On the other hand, if recruitment is detected in this way, P(high) should be decreased, T(high) or T(low) should be increased, or some combination thereof should be effected.

The present disclosure also contemplates in any of the embodiments an optimal end inspiratory lung volume (OEILV mode 1200 than can further augment aspects of the recruitment mode 400 of FIG. 6. In this alternative variation to any of the embodiments of the innovative ventilator and ventilator system 10, the OEILV mode 1200 is optionally or preferably invoked by the command module 30 as needed. More preferably, the OEILV mode 1200 is invoked by the recruitment mode 400. Even more preferably, the OEILV mode 1200 is invoked by the recruitment mode 400 at any moment outside the actual recruitment phases or inspiratory pressurization because the OEILV mode 1200 ideally assesses for derecruitment and is active or engaged only during the machine or ventilator 10 breath. Most preferably, the OEILV mode 1200 monitors the existing sensor data to identify changes in flow and time during the P(high)/T(high) cycle of ventilation.

When invoked, the OEILV mode 1200 is active over time during the machine breath and acquires recorded reference points of the flow/time course to P(high)/T(high) cycle. The OEILV mode 1200 uses this acquired data to identify changes in flow and time coordinate grid during the P(high)/T(high) cycle. If such changes are in fact identified, the OEILV mode 1200 may preferably communicate a message to the command module 30, the recruitment mode 400, and/or over the feedback loop 180, to initiate recruitment. Even more preferably, the OEILV mode 1200 may also suggest and/or effect manual or automated adjustments to P(high) and/or T(high) to further minimize actual or prospective derecruitment and/or to improve the pulmonary conditions of the ventilation therapy and/or the response or conditioning of the patient P.

In the non-recruitment phase of the recruitment mode 400, the OEILV mode 1200, when active, preferably may also intermittently adjust P(high), T(high), or both, and/or may notify the command module 30, the feedback loop 180, and/or other modes of the recommended adjustments, and/or may communicate to the clinician manually or automatically so as to seek clinical intervention if warranted. These adjustments in P(high), T(high), or both, may be applied in an occasional, intermittent, and/or cyclic manner, and may be effected either manually, through informative messages, or through automation.

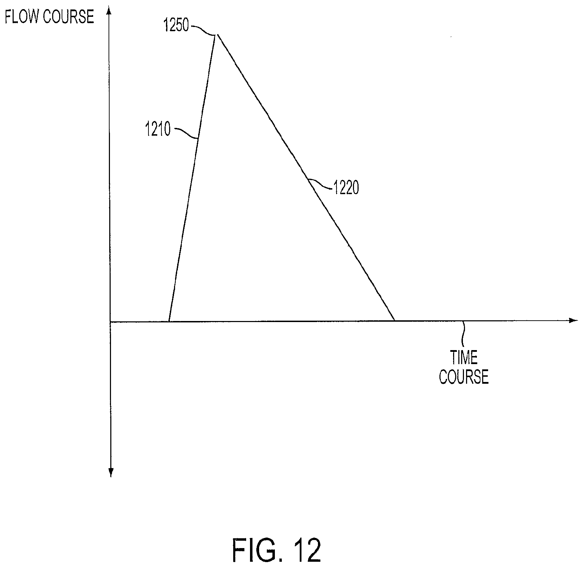

In further aspects of the optionally preferred OEILV mode 1200, and with reference also to FIG. 12, the OEILV mode 1200 may preferably incorporate a resistive element 1210 that occurs during the onset of the machine breath, an inflection point 1250 that correlates with an inflection or a half-way point of the machine breath cycle and an elastic element 1220 that corresponds with the relaxing subsequent to the machine breath. It is important to note that the OEILV mode 1200 measures the resistive-elastic transition point 1250 to determine if the slope of the elastic element 1220 changes.

In other words, the inquiry seeks to learn whether the elastic element 1220 becomes more acute in derecruitment and less acute in recruitment. Those skilled in the arts may come to understand that the combination of information available from FIGS. 11 and 12 and the accompanying discussion herein enables a heretofore unavailable means of more accurately discerning whether recruitment has been accomplished or whether derecruitment has occurred. The various modes now available enable more accurate and more automated systems for better managing and mitigating recruitment and derecruitment during many possible ventilation therapy protocols.

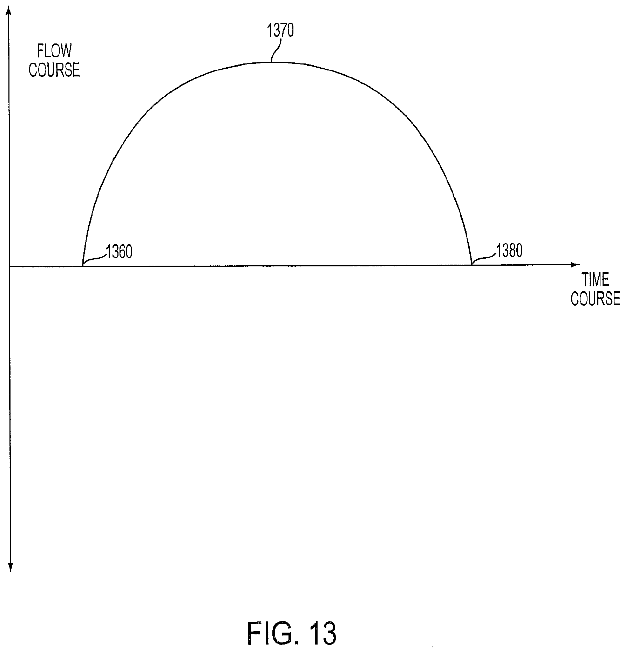

In any of the examples of the ventilator 10 and modes and methods of operation, the breathing spontaneity can be further assessed using an optionally preferred spontaneous breathing mode 1340 that is graphically depicted in FIG. 13. This spontaneous breathing mode 1340 may be further invoked by any of the other modes, modules, and routines of the inventive ventilator 10. Even so, this spontaneous breathing mode 1340 may find special utility in being optionally invoked through the command module 30 alone and/or by either the ventilation mode 500 or by the initial weaning mode 600.

In addition to comparing the actual spontaneous breaths per unit time of the patient P, this mode 1340 may preferably assess and analyze the nature of spontaneous breathing to identify and quantify breathing effort, otherwise referred to as the "work of breathing" More preferably, the spontaneous breathing mode 1340 assesses the effect of weaning on the work of breathing. Any of the sensors described elsewhere herein, such as one or more of the strain gauges 68, can be elastically or tightly affixed to the thorax of the patient P to sense and record movement, and solid-state or similarly capable accelerometers can also be used to gain additional data points that can be used to compute actual work expended to breathe.

Referring to FIG. 13 again, such data points can be correlated against a spontaneous breath initiation phase 1360, a spontaneous peak phase 1370, and a spontaneous termination phase 1380. Even more preferably, such data can be adduced during any of the spontaneous breath evaluations 570, 580 (FIGS. 7 and 8) occurring during the ventilation and initial weaning modes 500, 600, as well as any other suitable time. These additional indicia of the pulmonary conditioning and response of the patient P can further illuminate the patient's P true cardiopulmonary physiology, which can lessen the risk that the patient P is prematurely removed from ventilation therapy due to patient P resistance or other issues.

With continued reference to the various figures and preceding discussion, those knowledgeable in the relevant arts may appreciate that for certain preferred circumstances, the disclosure also contemplates initiating ventilation of the patient P in an APRV mode 700 based on initial oxygenation and ventilation settings. The patient P can then have the safety of the mandatory breath capability of the ventilator 10 while commencing ventilation therapy with a less intrusive profile. The ARPV airway pressure during expiration (P(low)) is substantially zero throughout ventilation to allow for the rapid acceleration of expiratory gas flow rates. Typically, the fraction of oxygen in the inspired gas (FiO₂) is initially set at about 0.5 to 1.0 (i.e., about 50% to 100%). The highest airway pressure achieved during inspiration (P(high)) must be sufficiently high to overcome airspace-closing forces and initiate recruitment of lung volume. P(high) may suitably be initialized at a default value of about 35 cm H₂O.

Alternatively, P(high) may be established based on either the severity and type of lung injury or the recruitment pressure requirements. The latter method is preferred in cases where the ratio of the partial pressure of oxygen in the blood of the patient P to the fraction of oxygen present in the inspired gas (i.e., PaO₂/FiO₂, which is commonly abbreviated as P/F) is less than or equal to about two hundred millimeters of mercury (200 mm Hg). The P/F ratio is preferably monitored continuously.

Where the type and severity of lung injury are characterized by a P/F of greater than about 350 mm Hg, an initial value of P(high) within the range of about 20 cm H₂O to 28 cm H₂O is preferably established. On the other hand, if the P/F ratio is less than about 350 mm Hg, P(high) is preferably initialized within the range of about 28 cm H₂O to 35 cm H₂O.

In situations where the P/F ratio is less than or equal to about 200 mm Hg, which may occur where the patient's P initial injury is non-pulmonary and/or the lung injury is of an indirect nature, the disclosure contemplates establishment of P(high) at a value of between about 35 mm Hg and 40 mm Hg but preferably not appreciably above 40 mm Hg. In cases where P(high) is initially established at a default value of about 35 cm H₂O, P(high) is reduced from such a value once P/F exceeds about 250 mm Hg. Initiation of ventilation also requires the establishment of time (duration) settings for inspiration and expiration.

Initially, the duration of the positive pressure phase (T(high)) is established at a value within the range of about 5.0 to about 6.0 seconds unless the measured PaCO2 is greater than about 60 mm Hg. In that case, T(high) is more preferably set to a lower initial value of within the range of about 4.0 to 5.0 seconds. The duration of the ventilator 10 release phase (T(low)) may suitably be initialized at a value within the range of 0.5 to 0.8 seconds with about 0.7 seconds being a preferred default value.

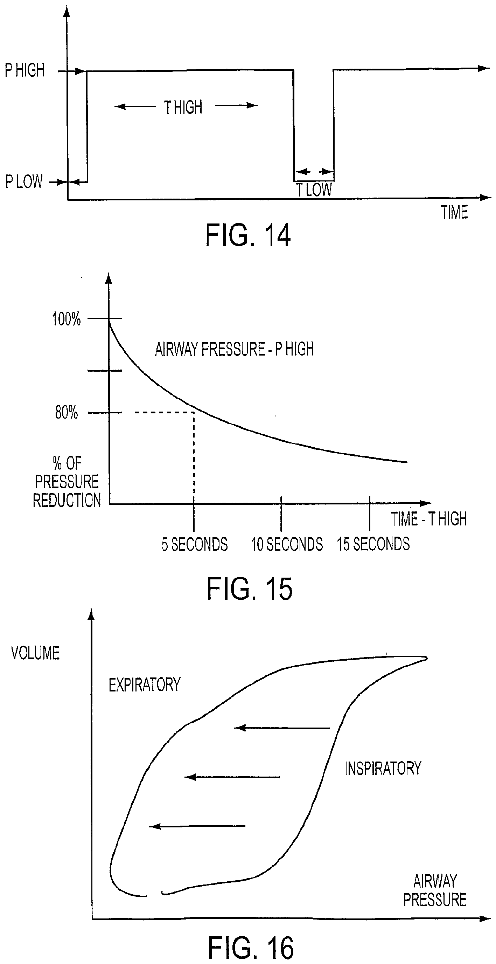

Once initial values of P(high), P(low), T(high) and T(low) have been established, ventilation continues in a repetitive APRV mode cycle generally as illustrated in FIG. 14. During management of ventilation in accordance with the disclosure, the initial values of one or more of these parameters are re-assessed and modified in accordance with measured parameters as has been described in connection with earlier descriptions.

In management of ventilation in accordance with the disclosure, a principal goal is to maintain the level of carbon dioxide in the blood of the ventilated patient P (PaCO₂) at a level of less than or equal to about 50 mm Hg. Toward that end, arterial PaCO₂ is monitored continuously or measured as clinically indicated and the ventilator 10 controlled to adjust ventilation as follows. Any time after ventilation has commenced, but preferably soon thereafter or promptly upon any indication of hypercarbia (PaCO₂ above about 50 mm Hg), the setting of T(low) is optionally but preferably checked and readjusted if necessary.

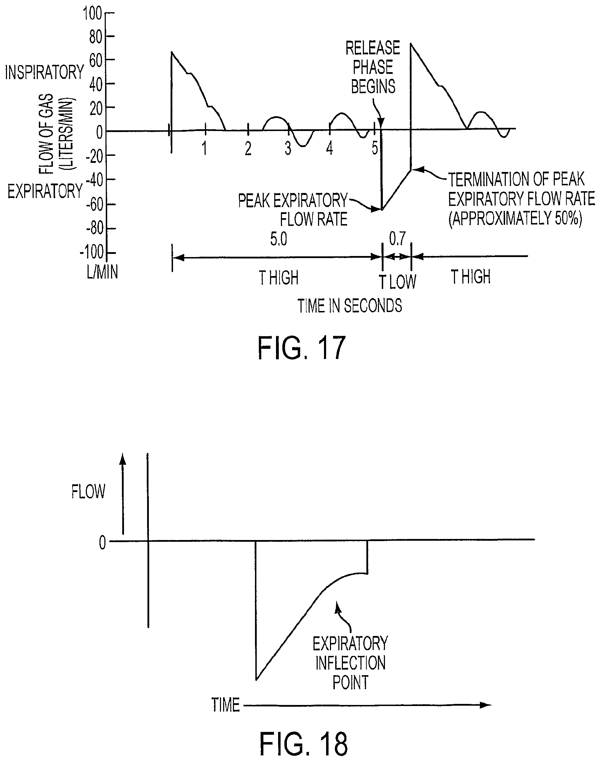

According to the disclosure, optimal end expiratory lung volume is maintained by titration of the duration of the expiration or release phase by terminating T(low) based on expiratory gas flow. To do so, the flow rate of the expiratory gas is measured by the ventilator 10 and checked in relation to the time at which the controller of the ventilator 10 initiates termination of the release phase. The expiratory exhaust valve should be actuated to terminate the release phase T(low), at a time when the flow rate of the expiratory gas has decreased to about 25% to 50% of its absolute peak expiratory flow rate (PEFR). An example is illustrated in FIG. 17. In that example, T(low) terminates by controlling the expiratory exhaust valve to terminate the release phase when the expiratory gas flow rate diminishes to 40% PEFR.

If monitoring of PaCO₂ indicates hypocarbia is present (i.e., PaCO₂ less than about 50 mm Hg), T(high) is increased by about 0.5 seconds while maintaining P(high) substantially unchanged. Should the patient P remain hypocarbic as indicated by subsequent measure of PaCO₂, weaning in the manner to be described may be initiated provided oxygenation is satisfactory and weaning is not otherwise contraindicated based on criteria to be described further below.

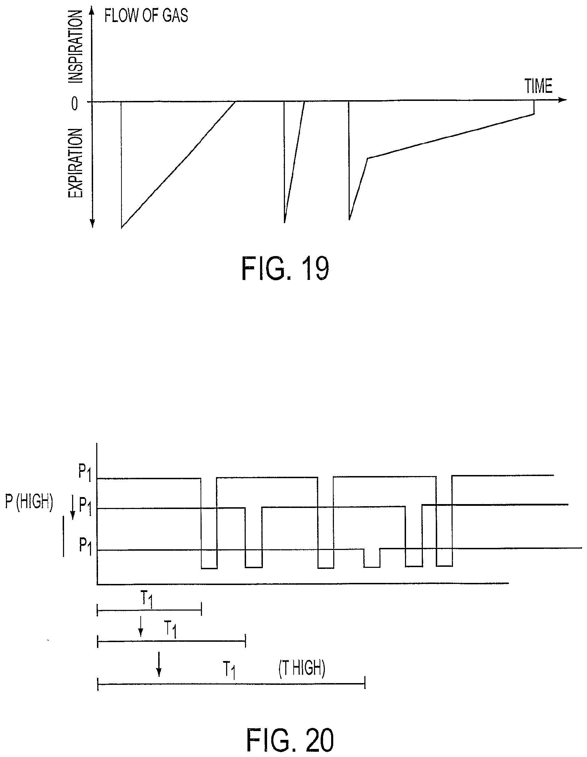

The hypercarbic patient P though is not to be weaned. In the event of hypercarbia, the disclosure contemplates assessment of the expiratory flow pattern before making significant further adjustments to ventilation parameters. This assessment can readily be carried out by a software program stored within the control unit of the ventilator 10, which carries out automated analysis of the expiration flow versus time tracing. As illustrated in FIG. 19, normal expiratory flow is characterized by flow that declines substantially monotonically from the onset of the release phase through its termination and does not fall off prematurely or abruptly. Restrictive flow in contrast declines rapidly from the onset of the release phase to zero or a relatively small value. Obstructive flow tends to be more extended in duration and is characterized by an inflection point beyond which the rate of flow falls off markedly from its initial rate.

FIG. 18 illustrates a gas flow pattern with a noticeable inflection point based on analysis of flow data provided by expiratory flow sensors, the control unit of the ventilator 10 is programmed to determine reference points during the P(low)/T(low) cycle. Flow and time reference points within the flow/time area, which is created or established by the P(low)/T(low) cycle, may be used to measure and calculate changes occurring in lung volume during the P(low)/T(low) cycle. If it is determined that obstructive or restrictive flow is present, the disclosure contemplates adjusting T(low) before making any other significant adjustments to ventilation parameters. This can be done according to either of two alternative methods.

One method is to adjust T(low) to a predetermined value according to whether flow is either obstructive or restrictive but allowing T(low) to remain at its previous value if flow is normal. In the case of restrictive flow, T(low) should be adjusted to less than about 0.7 seconds. On the other hand, obstructive flow calls for a T(low) of greater duration, preferably greater than about 0.7 seconds with 1.0 to 1.2 being typical.

It is optional but advisable to promptly assess the sedation level of the hypercarbic patient P. Sedation of the patient P can be evaluated by any suitable technique such as the conventional clinical technique of determining a sedation agitation scale (SAS) score for the patient P. If the patient P appears over-sedated based on the SAS score (SAS score greater than about 2) or otherwise, reduction of sedation should be considered and initiated if appropriate. Thereafter, T(high) should be increased by about 0.5 seconds and P(high) increased concomitantly by about 2 cm H₂O. After allowing sufficient time for these adjustments to take effect on the patient P, PaCO₂ should be re-evaluated. If the patient P remains hypercarbic, T(high) should be increased again by about 0.5 seconds and P(high) again increased concomitantly by about 2 cm H₂O. PaCO₂ should then be reassessed and concomitant increases of about 0.5 seconds in T(high) and about 2 cm H₂O in P(high) repeated until the patient P is no longer hypercarbic. However, the total duration of T(high) should not be increased beyond a maximum of about fifteen (15) seconds.

Management of oxygenation in accordance with the disclosure is carried out with the goal of maintaining the level of oxygen in the arterial blood of the ventilated patient P (PaO₂) at a value of at least about 80 mm Hg and a maintaining saturation level (SaO₂) of at least about 95%. Preferably, fluctuations of PaO₂ are held within a target range of about 55 mm Hg and 80 mm Hg. (Expressed in terms of SpO₂, the target range would be between about 0.88 and 0.95, although where PaO₂ and SpO₂ data are both available, PaO₂ would take precedence.) Responsive to a determination that oxygenation and saturation both meet the goals just specified, the ventilator 10 would be controlled to progressively decrease the fraction of oxygen in the inspired gas (FiO₂) by about 0.5 about every thirty (30) minutes to one (1) hour with the objective of maintaining a blood oxygen saturation level (SaO₂) of about 95% at a P(high) of about 35 cm H₂O and an FiO₂ of about 0.5. Upon meeting the latter objective, weaning in the manner to be described may be initiated provided the ventilation goal described earlier (i.e. a PaCO₂ of less than about 50 mm Hg) is met and weaning is not otherwise contraindicated.

However, if the goals of oxygenation of PaO₂ of at least about 80 mm Hg and arterial blood oxygen saturation (SaO₂) of at least about 95% cannot both be maintained at the then-current FiO₂, FiO₂ is not decreased. Instead, P(high) is increased to about 40 cm H₂O and T(high) increased substantially contemporaneously by about 0.5 seconds.

If such action does not result in raising oxygenation and saturation to at least the goals of PaO₂ of about 80 mm Hg and SaO₂ of about 95%, P(high) is increased to a maximum of about 45 cm H₂O and T(high) is progressively further increased by about 0.5 seconds to 1.0 seconds. Oxygenation and saturation are then re-evaluated and, if they remain below goal, FiO₂, if initially less than 1.0, may optionally be increased to about 1.0. Oxygen and saturation continue to be re-evaluated, and T(high) successively raised in increments of about 0.5 to 1.0 seconds until the stated oxygen and saturation goals are met.

Once those oxygenation and saturation goals are met, ventilation is controlled to maintain those goals while progressively decreasing FiO₂ and P(high) toward the levels at which initiation of weaning can be considered. More particularly, P(high) is decreased by about 1 cm H₂O per hour while FiO₂ is decreased by about 0.05 about every thirty (30) minutes while maintaining an oxygen saturation of at least about 95%.

Weaning according to the disclosure, unless otherwise contraindicated, may commence after the oxygenation and ventilation goals described above have been met. That is, when PaCO₂ remains below about 50 mm Hg and SaO₂ remains at least about 95% at a P(high) of about 35 cm H₂O and FiO₂, if previously higher, has been weaned to a level of not greater than about 0.5. During weaning in accordance with the disclosure, T(high) is controlled to sustain recruitment while P(high) is reduced to gradually reduce airway pressure. As FIG. 20 illustrates, this is achieved by carrying out a series of successive incremental reductions in P(high) while substantially contemporaneously carrying out a series of successive incremental increases in T(high) so as to induce gradual pulmonary stress relaxation as FIG. 15 illustrates.

As a result, the pulmonary pressure versus volume curve shifts progressively from its inspiratory limb to its expiratory limb as illustrated in FIG. 16. As can be understood with reference to the previously described figures, weaning may be carried out in two stages, the first of which is more gradual than the second. During the first stage, P(high) is reduced by about 2 cm H₂O about every hour. Substantially contemporaneously with each reduction in P(high), T(high) is increased by about 0.5 to 1.0 seconds up to, but not in excess of a T(high) of about 15 seconds in total duration.

As P(high) is being reduced in the manner just described, the fraction of oxygen in the inspired gas (FiO₂) is also gradually reduced in accordance with P(high). During the first stage of weaning, this gradual weaning of FiO₂ is carried out gradually. When P(high) has been reduced to about 24 cm H₂O and FiO₂ weaned to about 0.4 with the patient P sustaining a blood oxygen saturation (SaO₂) of at least about 95% weaning may proceed to the more aggressive second stage. The term "substantially contemporaneously" should not be construed to be limited to necessarily require that changes occur precisely at the same moment. Rather, the term is to be construed broadly to encompass not merely events that occur at the same time, but also any which are close enough in time to achieve the advantages or effects described.

During continued weaning, successive reductions in P(high) and substantially contemporaneous increases in T(high) contemporaneous reductions continue about once every hour. However, during the second stage, the reductions in P(high) take place in increments of about 4 cm H₂O and the increases in T(high) are each about 2.0 seconds. As reductions in P(high) continue, further weaning of FiO₂ is implemented. Once FiO₂ is weaned to about 0.3, airway pressures are reduced such that the ventilation mode by then has been transitioned from APRV to a substantially Continuous Positive Airway Pressure/Automatic Tube Compensation Mode (CPAP/ATC).

Once the patient P is tolerating CPAP at about 5 cm H₂O with FiO₂ of not greater than about 0.5, the patient's P ability to maintain unassisted breathing is assessed, preferably for at least about two (2) hours or more. Criteria for such assessments include: a) SpO₂ of at least about 0.90 and/or PaO₂ of at least about 60 mm Hg; b) tidal volume of not less than about 4 ml/kg of ideal bodyweight; c) respiration rate not significantly above about 35 breaths per minute, and d) lack of respiratory distress, with such distress being indicated by the presence of any two or more of the following: i) Heart rate greater than 120% of the 0600-hour rate (though less than about five (5) minutes above such rate may be considered acceptable) ii) marked use of accessory muscles to assist breathing; iii) thoroco-abdominal paradox; iv) diaphoresis and/or v) marked subjective dyspnea.

If there is an indication of respiratory distress, CPAP at an airway pressure of about 10 cm H₂O should be resumed and monitoring and reassessment carried out as needed. However, if criteria a) through d) above are all satisfied, the patient P may be transitioned to substantially unassisted breathing such as by extubation with face mask, nasal prong oxygen or room air, T-tube breathing, tracheotomy mask breathing or use of high flow CPAP at about 5 cm H₂O.

During all phases of ventilation, including initiation, management and weaning, the patient P should be reassessed at least about every two (2) hours and more frequently if indicated. Blood gas measurements (PaO₂, SaO₂ and PaCO₂) which govern control of ventilation according to the disclosure should be monitored not less frequently than every two (2) hours though substantially continuous monitoring of all parameters would be ideal.

Just prior to and during weaning at least one special assessment should be conducted daily, preferably between 0600 and 1000 hours. If not possible to do so, a delay of not more than about four (4) hours could be tolerated. Weaning should not be initiated or continued further unless: (a) at least about 12 hours have passed since initial ventilation settings were established or first changed, (b) the patient P is not receiving neuromuscular blocking agents and is without neuromuscular blockade, and (c) Systolic arterial pressure is at least about 90 mm Hg without vasopressors (other than "renal" dose dopamine).

If these criteria are all met, a trial should be conducted by ventilating the patient P in CPAP mode at about 5 cm H₂O and an FiO₂ of about 0.5 for about five (5) minutes. If the respiration rate of the patient P does not exceed about 35 breaths per minute (bpm) during the five (5) minute period weaning as described above may proceed. However, if during the five (5) minute period the respiration rate exceeds about 35 bpm, it should be determined whether such tachypnea is associated with anxiety. If so, administer appropriate treatment for the anxiety and repeat the trial within about four (4) hours. If tachypnea does not appear to be associated with anxiety, resume management of ventilation at the parameter settings in effect prior to the trial and resume management of ventilation as described above. Re-assess at least daily until weaning as described above can be initiated.

### INDUSTRIAL APPLICABILITY

The examples of the present disclosure are suitable for use in many respiratory assistance applications that involve the use of ventilators and ventilator systems and methods of operation thereof. The various configurations and capabilities of the ventilator and system and method of operation can be modified to accommodate nearly any conceivable respiratory assistance application and/or requirement. The arrangement, capability, and compatibility of the features and components of the novel ventilators, systems, and methods of operation and use described herein can be readily modified according to the principles of the invention as may be required to suit any particular critical and/or routine care and/or hospital, assisted care, or home care application or situation. Additionally, such inventive ventilators, systems, and methods are suitable for use with nearly all types of ventilation equipment including but not limited to positive pressure or negative pressure respiratory assistance devices.

Such modifications and alternative arrangements may be further preferred and/or optionally desired to establish compatibility with the wide variety of possible applications that are susceptible for use with the improved ventilators, respiratory assistance systems, and operational methods that are described and contemplated herein. Accordingly, even though only few such embodiments, alternatives, variations, and modifications of the present invention are described and illustrated, it is to be understood that the practice of such additional modifications and variations and the equivalents thereof, are within the scope of the invention as defined in the following claims.

## Claims

1. A ventilator system (10) for assisting the respiratory function of a patient under the direction of a clinician, comprising:
a supply pump (12) and a control module (20) in communication with a data circuit (22) and a gas circuit (40) having a plurality of valves (50, 52) and supply (42) and exhaust (44) ports, the control module including a display (26), input device (28), and a memory (24) in communication with the data circuit;
a sensor array (54) in communication with the data circuit that includes at least one oximeter (56), at least one capnometer (58), at least one pressure sensor (64), and at least one flow meter in communication with at least one of the supply and exhaust ports;
a command module (30) resident in the memory operative to command the control module to adjustably actuate the pump and the plurality of valves to establish at least one pressure, volume or flow rate in the gas circuit;
at least one initialization parameter database resident in the memory to be communicable with the display and configured to store at least one model patient data array element that includes at least an FiO2 quantity, a high pressure, a low pressure, a high time, and a low time;
wherein the command module is configured to receive settings from the clinician via the input device and is configured to command the control module to actuate the supply pump and commence respiratory assistance to the patient whereby the gas circuit communicates with the patient using one each of the FiO2 quantity, the high and low pressure, and the high and low time;
wherein the command module is configured to communicate with the sensor array to measure a patient actual data array elements of at least one of (i) a patient SpO2 quantity, (ii) a patient etCO2 quantity, (iii) a peak expiratory flow rate, (iv) an end expiratory lung volume and (v) a spontaneous breathing frequency;
wherein the command module comprises an initial setup module (100) with an optimal end expiratory lung volume assessment mode (150) configured to monitor a number of key patient parameters to ascertain and periodically compute an optimal end expiratory lung volume, an adjustment and maintenance module (200) with an oxygenation mode (300), a recruitment mode (400) and a ventilation mode (500), and a weaning module (250), and
wherein the command module is configured to compare the patient actual data array to the at least one model patient data array and compute at least one of an SpO2 goal value, an etCO2 goal value, and the optimal end expiratory lung volume, which SpO2 goal value, etCO2 goal value and optimal end expiratory lung volume are used to determine whether the patient should be initially weaned, undergo recruitment and increased oxygenation, or be maintained in an unaltered state of ventilation to achieve the SpO2 goal value, the etCO2 goal value, and the optimal end expiratory lung volume.

2. The ventilator system according to claim 1, wherein the weaning module (250) comprises an initial weaning protocol (600), an airway pressure release ventilation protocol mode (700), and a continuous airway pressure or CPAP protocol mode (800).

3. The ventilator system according to claim 1, wherein the at least one model patient data array elements further includes at least one of a positive end expiratory pressure, an SpO2 quantity, an etCO2 quantity, a pressure increment, a time increment, a tidal volume, a machine respiratory frequency, a pressure-volume slope, a trigger pressure, and occlusion pressure.

4. The ventilator system according to Claim 1, wherein if the SpO2 goal value is false, the command module is configured to communicate with the sensor array and ascertain the patient actual data array to ascertain the high pressure value; and
wherein when the high pressure value is true the command module is configured to determine a FiO2 goal value that (a) when true, the control module adjusts at least one of the supply pump and the plurality of valves to increase the high pressure by a pressure increment and to increase the high time by a time increment, and (b) when false, the control module adjusts at least one of the supply pump and the plurality of valves to increase the FiO2 quantity.

5. The ventilator system according to Claim 1, wherein if the SpO2 goal value is true, the command module is configured to communicate with the sensor array and ascertain the patient actual data array to ascertain a FiO2 goal value; and
wherein when the FiO2 goal value (a) is true, the command module is configured to command the control module to adjust at least one of the supply pump and the plurality of valves to decrease the FiO2 quantity, and (b) when false, the command module is configured to set an initial weaning value to be true.

6. The ventilator system according to Claim 1, wherein if the SpO2 goal value is false, the command module is configured to communicate with the sensor array and ascertain the patient actual data array to compute a recruitment value; and
wherein when the recruitment value (i) is true, the control module is configured to generate a clinician alarm signal, and, (ii) is false the control module is configured to adjust at least one of the supply pump and the plurality of valves to (a) increase the high pressure by a pressure increment, (b) increase the high time by a time increment, and (c) adjust the low time by another time increment.

7. The ventilator system according to Claim 1, wherein if the SpO2 goal value is false, the command module is configured to communicate with the sensor array and ascertain the patient actual data array to compute an optimal end expiration lung volume value; and
wherein if the optimal end expiration lung volume value (a) is true, the command module is configured to set an oxygenation value to be true, and (b) if false, the command module is configured to (i) poll the sensor array to measure the peak expiratory flow rate, measure the truncation of gas flow, and to compute an angle of deceleration of gas flow to select one of the time increments, (ii) set a recruitment value to be true, and (iii) command the control module to adjust at least one of the supply pump and the plurality of valves to decrease the low time by applying the selected time increment.

8. The ventilator system according to Claim 3, wherein the command module is configured to determine the high pressure if the etCO2 goal value, the comparison between the spontaneous frequency and the machine respiratory frequency and the high time is false; and
wherein the command module is configured to command the control module to adjust at least one of the supply pump and the plurality of valves by increasing at least one of the high time and the high pressure by at least one respective time increment and pressure increment if the high pressure is determined to be false, and wherein the command module is configured to command the control module to adjust at least one of the supply pump and the plurality of valves by increasing at least one of the high time by at least one respective time increment if the high pressure is determined to be true.

9. The ventilator system according to Claim 3, wherein the command module is configured to determine the high pressure if the etCO2 goal value is false, the comparison between spontaneous frequency and the machine respiratory frequency is true and the high time is true; and
wherein the command module is configured to command the control module to set a recruitment value to be true and to adjust at least one of the supply pump and the plurality of valves by changing the high time by at least one respective time increment if the high pressure is determined to be true, and wherein the command module is configured to command the control module to adjust at least one of the supply pump and the plurality of valves by decreasing the high time and increasing the high pressure by at least one respective time increment and pressure increment if the high pressure is determined to be false.

10. The ventilator system according to Claim 3, wherein the command module is configured such that when the control module detects the etCO2 goal value to be true and the command module samples to ascertain a spontaneous frequency:
a) when the spontaneous frequency is false, the command module ascertains a tachypnea value that if true enables the command module to set a ventilation value to be true, and
b) when the spontaneous frequency is true, the command module ascertains an apnea value that (i) when true enables the command module to set the ventilation value to be true and (ii) when false enable the command module to set an airway pressure release ventilation weaning value to be true.

11. The ventilator system according to Claim 3, wherein the command module is configured such that when the control module detects the etCO2 goal value to be true and the command module samples the high pressure and sets a high pressure value and samples the spontaneous frequency, and when the command module detects the spontaneous frequency and the high pressure value are true, the command module initiates a weaning by commanding the control module to adjust at least one of the supply pump and the plurality of valves to decrease the high pressure by a pressure increment and to increase the high time by a time increment.

12. The ventilator system according to Claim 3, further comprising:
the at least one model patient data array further including predetermined weaning failure criteria that establish a FiO2 threshold, a SpO2 threshold, a spontaneous tidal volume, a minute ventilation quantity, and an airway occlusion pressure;
wherein the command module is configured to communicate with the data circuit to sample the sensor array and measure at least one of the patient actual data array elements and to compare the elements to the predetermined weaning failure criteria to generate a weaning failure value; and
wherein the command module is configured such that when the command module determines (a) that the weaning failure value is true, the command module commands the control module to adjust at least one of the supply pump and the plurality of valves to increase the high pressure by a pressure increment and to decrease the high time by a time increment, and such that when the command module determines (b) that the weaning failure value is false, the command module repeatedly initiates cyclic weaning by commanding the control module to adjust at least one of the supply pump and the plurality of valves to decrease the high pressure and high time by a pressure and time increment.

13. The ventilator system according to Claim 12, wherein the command module is configured such that each time the command module initiates another cyclic weaning, the command module ascertains the high pressure until a continuous positive airway pressure threshold is reached to enable the command module to set a continuous positive airway pressure value to be true.

14. The ventilator system according to Claim 3, further comprising:
the at least one model patient data array further including a predetermined continuous positive airway pressure and a predetermined weaning failure criteria establishing a FiO2 threshold, a SpO2 threshold, a spontaneous tidal volume, a minute ventilation quantity, and an airway occlusion pressure;
wherein the command module is configured to communicate with the data circuit to sample the sensor array and measure at least one of the patient actual data array elements and to compare the elements to the predetermined weaning failure criteria to generate a weaning failure value; and
wherein the command module is configured such that when the command module determines (a) that the weaning failure value is true, the command module commands the control module to adjust at least one of the supply pump and the plurality of valves to increase the continuous positive airway pressure, and such that when the command module determines (b) that the weaning failure value is false, the command module periodically decreases the continuous positive airway pressure until an extubate threshold pressure is reached.

15. The ventilator system according to Claim 14,
wherein the command module is configured such that when the high pressure is (a) false, the command module commands the control module to adjust at least one of the supply pump and the plurality of valves to adjust the continuous positive airway pressure based on the high pressure, and such that when the high pressure is (b) true, the command module commands the control module to adjust at least one of the supply pump and the plurality of valves to adjust to airway pressure release ventilation weaning.

## Patentansprüche

1. Beatmungssystem (10) zur Unterstützung der Atemfunktion eines Patienten unter der Leitung eines Klinikers, umfassend:
eine Zufuhrpumpe (12) und ein Steuermodul (20) in Verbindung mit einem Datenschaltkreis (22) und einem Gaskreislauf (40) mit mehreren Ventilen (50, 52) und Versorgungs- (42) und Auslassöffnungen (44), wobei das Steuermodul eine Anzeige (26), eine Eingabevorrichtung (28) und einen Speicher (24) in Kommunikation mit der Datenschaltkreis enthält;
ein Sensorarray (54), das mit der Datenschaltkreis in Verbindung steht, das mindestens ein Oximeter (56), mindestens ein Kapnometer (58), mindestens einen Drucksensor (64) und mindestens einen Durchflussmesser in Verbindung mit mindestens einer der Zufuhr- und Auslassöffnungen enthält;
ein in dem Speicher residentes Befehlsmodul (30), das betreibbar ist, um dem Steuermodul zu befehlen, die Pumpe und die Vielzahl von Ventilen einstellbar zu betätigen, um mindestens einen Druck, ein Volumen oder eine Durchflussrate in dem Gaskreislauf einzurichten;
mindestens eine Initialisierungsparameterdatenbank, die im Speicher resident ist, um mit der Anzeige kommunizierbar zu sein und konfiguriert ist, um mindestens ein Modellpatientendatenarrayelement zu speichern, das mindestens eine FiO2-Menge, einen Hochdruck, einen Niederdruck, eine hohe Zeit und eine niedrige Zeit enthält;
wobei das Befehlsmodul konfiguriert ist, um Einstellungen von dem Kliniker über das Eingabegerät zu empfangen, und konfiguriert ist, um dem Steuermodul zu befehlen, die Zufuhrpumpe zu betätigen und die Atemunterstützung für den Patienten zu beginnen, wobei der Gaskreislauf mit dem Patienten unter Verwendung von jeweils einem der FiO2 Menge, der Hoch- und Niederdruck, und die hohe und niedrige Zeit kommuniziert;
wobei das Befehlsmodul konfiguriert ist, um mit dem Sensorarray zu kommunizieren, um Elemente eines tatsächliche Patientendatenarray von mindestens einem von (i) einer SpO2-Menge des Patienten, (ii) einer etCO2-Menge des Patienten, (iii) einer exspiratorischen Spitzenflussrate, (iv) ein endexspiratorisches Lungenvolumen und (v) eine spontane Atemfrequenz zu messen;
wobei das Befehlsmodul ein anfängliches Setup-Modul (100) mit einem optimalen endexspiratorischen Lungenvolumen-Bewertungsmodus (150) umfasst, der konfiguriert ist, um eine Reihe von Schlüsselpatientenparametern zu überwachen, um ein optimales endexspiratorisches Lungenvolumen zu ermitteln und periodisch zu berechnen, ein Anpassungs- und Wartungsmodul (200) mit einem Oxygenierungsmodus (300), einem Rekrutierungsmodus (400) und einem Beatmungsmodus (500) und einem Entwöhnungsmodul (250), und
wobei das Befehlsmodul konfiguriert ist, um das tatsächliche Patientendatenarray mit dem mindestens einen Modellpatientendatenarray zu vergleichen und mindestens einen von einem SpO2-Zielwert, einem etCO2-Zielwert und dem optimalen endexspiratorischen Lungenvolumen zu berechnen, wobei der SpO2-Zielwert, etCO2-Zielwert und optimales endexspiratorisches Lungenvolumen werden verwendet, um zu bestimmen, ob der Patient anfänglich entwöhnt, rekrutiert und mit erhöhter Oxygenierung behandelt oder in einem unveränderten Beatmungszustand gehalten werden sollte, um den SpO2-Zielwert, den etCO2-Zielwert und das optimale endexspiratorische Lungenvolume zu erreichen.

2. Beatmungssystem nach Anspruch 1, wobei das Entwöhnungsmodul (250) ein anfängliches Entwöhnungsprotokoll (600), einen Atemwegsdruckfreigabe-Beatmungsprotokollmodus (700) und einen kontinuierlichen Atemwegsdruck- oder CPAP-Protokollmodus (800) umfasst.

3. Beatmungssystem nach Anspruch 1, wobei das mindestens eine
Modellpatientendatenarrayelement ferner mindestens eines von einem positiven endexspiratorischen Druck, einer SpO2-Menge, einer etCO2-Menge, einem Druckinkrement, einem Zeitinkrement, einem Tidalvolumen, eine Atemfrequenz der Maschine, eine DruckVolumen-Steigung, einen Triggerdruck und einen Okklusionsdruck enthält.

4. Beatmungssystem nach Anspruch 1, wobei, wenn der SpO2-Zielwert falsch ist, das Befehlsmodul konfiguriert ist, um mit dem Sensorarray zu kommunizieren un das tatsächliche Patientendatenarray zu ermitteln, um den Hochdruckwert zu ermitteln; und
wobei, wenn der Hochdruckwert wahr ist, das Befehlsmodul konfiguriert ist, um einen FiO2-Zielwert zu bestimmen, der (a) wenn er wahr ist, das Steuermodul die Zufuhrpumpe und/oder die Vielzahl von Ventilen einstellt, um den Hochdruck um einen Druckinkrement zu erhöhen und die hohe Zeit um ein Zeitinkrement zu erhöhen, und (b) wenn falsch, stellt das Steuermodul mindestens eines von der Zufuhrpumpe und der Vielzahl von Ventilen ein, um die FiO2-Menge zu erhöhen.

5. Beatmungssystem nach Anspruch 1, wobei, wenn der SpO2-Zielwert wahr ist, das Befehlsmodul konfiguriert ist, um mit dem Sensorarray zu kommunizieren und das tatsächliche Patientendatenarray zu ermitteln, um einen FiO2-Zielwert zu ermitteln; und
wobei, wenn der FiO2-Zielwert (a) wahr ist, das Befehlsmodul konfiguriert ist, um dem Steuermodul zu befehlen, die Zufuhrpumpe und/oder die Vielzahl von Ventilen einzustellen, um die FiO2-Menge zu verringern, und (b) wenn falsch, das Befehlsmodul konfiguriert ist um ein anfänglicher Entwöhnungswert auf wahr zu setzen.

6. Beatmungssystem nach Anspruch 1, wobei, wenn der SpO2-Zielwert falsch ist, das Befehlsmodul konfiguriert ist, um mit dem Sensorarray zu kommunizieren und das tatsächliche Patientendatenarray zu ermitteln, um einen Rekrutierungswert zu berechnen; und
wobei, wenn der Rekrutierungswert (i) wahr ist, das Steuermodul konfiguriert ist, um ein Kliniker-Alarmsignal zu erzeugen, und (ii) falsch ist, das Steuermodul konfiguriert ist, um mindestens eines von der Zufuhrpumpe und der Vielzahl von Ventilen so einzustellen, dass (a) den Hochdruck um ein Druckinkrement zu erhöhen, (b) die hohe Zeit um ein Zeitinkrement zu erhöhen und (c) die niedrige Zeit um ein weiteres Zeitinkrement an zu passen.

7. Beatmungssystem nach Anspruch 1, wobei, wenn der SpO2-Zielwert falsch ist, das Befehlsmodul konfiguriert ist, um mit dem Sensorarray zu kommunizieren und das tatsächliche Patientendatenarray zu ermitteln, um einen optimalen Lungenvolumenwert am End der Exspiration zu berechnen; und
wobei, wenn der optimale Lungenvolumenwert am Ende der Exspiration (a) wahr ist, das Befehlsmodul konfiguriert ist, um einen Oxygenierungswert auf wahr zu setzen, und (b) falls falsch, das Befehlsmodul konfiguriert ist, (i) das Sensorarray abzufragen, um Messen der maximalen Exspirationsflussrate, Messen des Abschneidens des Gasflusses und Berechnen eines Verlangsamungswinkels des Gasflusses, um eines der Zeitinkremente auszuwählen, (ii) einen Einstellungswert als wahr zu setzen und (iii) die Steuerungsmodul zu befehlen, um mindestens eines von der Zufuhrpumpe und der Vielzahl von Ventilen einzustellen, um die niedrige Zeit durch Anwenden des ausgewählten Zeitinkrements zu verringern.

8. Beatmungssystem nach Anspruch 3, wobei das Befehlsmodul konfiguriert ist, um den Hochdruck zu bestimmen, wenn der etCO2-Zielwert, der Vergleich zwischen der spontanen Frequenz und der Maschinenatemfrequenz und der hohen Zeit falsch ist; und
wobei das Befehlsmodul konfiguriert ist, um dem Steuermodul zu befehlen, mindestens eines von der Zufuhrpumpe und der Vielzahl von Ventilen einzustellen, indem mindestens eines von der hohen Zeit und dem Hochdruck um mindestens ein jeweiliges Zeitinkrement und Druckinkrement erhöht wird, wenn die Hochdruck als falsch bestimmt wird, und wobei das Befehlsmodul konfiguriert ist, um dem Steuermodul zu befehlen, mindestens eines von der Zufuhrpumpe und der Vielzahl von Ventilen einzustellen, indem mindestens eine von der hohen Zeit um mindestens ein jeweiliges Zeitinkrement erhöht wird wenn festgestellt wird, dass der Hochdruck wahr ist.

9. Beatmungssystem nach Anspruch 3, wobei das Befehlsmodul konfiguriert, um den Hochdruck zu bestimmen, wenn der etCO2-Zielwert falsch ist, der Vergleich zwischen der spontanen Frequenz und der Maschinenatemfrequenz wahr ist und der hohe Zeit wahr ist; und
wobei das Befehlsmodul konfiguriert ist, um dem Steuermodul zu befehlen, einen Einstellungswert auf wahr zu setzen und mindestens eines von der Zufuhrpumpe und der Vielzahl von Ventilen durch Ändern der hohe Zeit um mindestens ein jeweiliges Zeitinkrement einzustellen, wenn der Hochdruck als wahr bestimmt wird, und wobei das Befehlsmodul konfiguriert ist, um dem Steuermodul zu befehlen, mindestens eines von der Zufuhrpumpe und der Vielzahl von Ventilen durch Verringern der Hohe Zeit und Erhöhen des Hochdrucks um mindestens ein jeweiliges Zeitinkrement einzustellen und Druckinkrement, wenn festgestellt wird, dass der Hochdruck falsch ist.

10. Beatmungssystem nach Anspruch 3, wobei das Befehlsmodul so konfiguriert ist, dass, wenn das Steuermodul erkennt, dass der etCO2-Zielwert wahr ist und das Befehlsmodul abtastet, um eine spontane Frequenz zu ermitteln; und
a) wenn die spontane Frequenz falsch ist, ermittelt das Befehlsmodul einnen Tachypnoe-Wert, der, wenn er wahr ist, dem Befehlsmodul ermöglicht, einen Beatmungswert auf wahr zu setzen, und
b) wenn die spontane Frequenz wahr ist, ermittelt das Befehlsmodul einen Apnoewert, der (i) wenn wahr das Befehlsmodul ermöglicht, den Beatmungswert auf wahr zu setzen, und (ii) wenn falsch, das Befehlsmodul aktiviert, eine Atemwegsdruckfreigabe enzustellen Beatmungsentwöhnungswert ist wahr.

11. Beatmungssystem nach Anspruch 3, wobei das Befehlsmodul so konfiguriert ist, dass, wenn das Steuermodul erkennt, dass der etCO2-Zielwert wahr ist und das Befehlsmodul den Hochdruck abtastet und einen Hochdruckwert einstellt und die spontane Frequenz abstatet, und wenn das Befehlsmodul erkennt, dass die spontane Frequenz und der Hochdruckwert wahr sind, das Befehlsmodul eine Entwöhnung einleitet, indem es dem Steuermodul befiehlt, die Zufuhrpumpe und/oder die Mehrzahl von Ventilen einzustellen, um den Hochdruck um ein Druckinkrement zu verringern und um die hohe Zeit um ein Zeitinkrement zu erhöhen.

12. Beatmungssystem nach Anspruch 3, ferner umfassend:
wobei das mindestens eine Modellpatientendatenarray ferner vorbestimmte Entwöhnungsversagenskriterien enthält, die einen FiO2-Schwellenwert, einen SpO2-Schwellenwert, ein spontanes Atemzugvolumen, eine Minutenventilationsmenge und einen Atemwegsverschlussdruck festlegen;
wobei das Befehlsmodul konfiguriert ist, um mit der Datenschaltung zu kommunizieren, um das Sensorarray abzutasten und mindestens eines der Elemente des tatsächlichen Patientendatenarrays zu messen und die Elemente mit den vorbestimmten Entwöhnungsfehlerkriterien zu vergleichen, um einen Entwöhnungsfehlerwert zu erzeugen; und
wobei das Befehlsmodul so konfiguriert ist, dass, wenn das Befehlsmodul bestimmt (a), dass der Entwöhnungsfehlerwert wahr ist, das Befehlsmodul das Steuermodul befiehlt, die Zufuhrpumpe und/oder die Mehrzahl von Ventilen einzustellen, um den Hochdruck zu erhöhen um ein Druckinkrement und zum Verringern der hohen Zeit um ein Zeitinkrement, und so dass, wenn das Befehlsmodul feststellt (b), dass der Entwöhnungsfehlerwert falsch ist, das Befehlsmodul wiederholt die zyklische Entwöhnung einleitet, indem es dem Steuermodul befiehlt, mindestens eines von der Zufuhrpumpe oder der Vielzahl von Ventilen, um den Hochdruck und die hohe Zeit um ein Druck- und Zeitinkrement zu verringern.

13. Beatmungssystem nach Anspruch 12, wobei das Befehlsmodul so konfiguriert ist, dass jedes Mal, wenn das Befehlsmodul eine weitere zyklische Entwöhnung einleitet, das Befehlsmodul den Hochdruck ermittelt, bis ein kontinuierlicher positiver Atemwegsdruckschwellenwert erreicht ist, um es dem Befehlsmodul zu ermöglichen einen kontinuierlichen positiven Atemwegdruckwert auf wahr setzen kann.

14. Beatmungssystem nach Anspruch 3, ferner umfassend:
wobei das mindestens eine Modellpatientendatenarray ferner einen vorbestimmten kontinuierlichen positiven Atemwegsdruck und ein vorbestimmtes Entwöhnungsversagenskriterium umfasst, das einen FiO2-Schwellenwert, einen SpO2-Schwellenwert, ein spontanes Atemzugvolumen, eine Minutenventilationsmenge und einen Atemwegsverschlussdruck festlegt;
wobei das Befehlsmodul konfiguriert ist, um mit der Datenschaltung zu kommunizieren, um das Sensorarray abzutasten und mindestens eines der Elemente des tatsächlichen Patientendatenarrays zu messen und die Elemente mit den vorbestimmten Entwöhnungsfehlerkriterien zu vergleichen, um einen Entwöhnungsfehlerwert zu erzeugen; und
wobei das Befehlsmodul so konfiguriert ist, dass, wenn das Befehlsmodul bestimmt (a), dass der Entwöhnungsfehlerwert wahr ist, das Befehlsmodul das Steuermodul befiehlt, die Zufuhrpumpe und/oder die Mehrzahl von Ventilen einzustellen, um den kontinuierlichen positiven Atemwegsdruck zu erhöhen, und so dass, wenn das Befehlsmodul (b) feststellt, dass der Entwöhnungsfehlerwert falsch ist, das Befehlsmodul den kontinuierlich positiven Atemwegsdruck periodisch verringert, bis ein Extubationsschwellendruck erreicht ist.

15. Beatmungssystem nach Anspruch 14,
wobei das Befehlsmodul so konfiguriert ist, dass, wenn der Hochdruck (a) falsch ist, das Befehlsmodul dem Steuermodul befiehlt, die Zufuhrpumpe und/oder die Vielzahl von Ventilen einzustellen, um den kontinuierlichen positiven Atemwegsdruck basierend auf dem Hochdruck einzustellen, und derart, dass, wenn der Hochdruck (b) wahr ist, das Befehlsmodul dem Steuermodul befiehlt, mindestens eines von der Zufuhrpumpe und der Vielzahl von Ventilen einzustellen, um die Beatmungsentwöhnung mit Atemwegsdruckfreigabe einzustellen.

## Revendications

1. Un système de ventilation (10) pour assister la fonction respiratoire d'un patient sous la direction d'un clinicien, comprenant :
une pompe d'approvisionnement (12) et un module de commande (20) en communication avec un circuit de données (22) et un circuit de gaz (40) ayant une pluralité de vannes (50, 52) et des orifices d'approvisionnement (42) et d'échappement (44), le module de commande comprenant un affichage (26), un dispositif d'entrée (28) et une mémoire (24) en communication avec le circuit de données ;
un tableau de capteurs (54) en communication avec le circuit de données qui comprend au moins un oxymètre (56), au moins un capnomètre (58), au moins un capteur de pression (64) et au moins un débitmètre en communication avec au moins l'un des orifices d'approvisionnement et d'échappement ;
un module de commande (30) résidant dans la mémoire fonctionnant pour commander au module de commande d'actionner de manière réglable la pompe et la pluralité de vannes pour établir au moins une pression, un volume ou un débit dans le circuit de gaz ;
au moins une base de données de paramètres d'initialisation résidant dans la mémoire pour pouvoir communiquer avec l'affichage et configurée pour stocker au moins un élément de tableau de données de patient modèle qui comprend au moins une quantité de FiO2, une pression haute, une pression basse, un temps haute et un temps bas ;
dans lequel le module de commande est configuré pour recevoir des réglages du clinicien via le dispositif d'entrée et est configuré pour commander au module de commande d'actionner la pompe d'approvisionnement et de commencer l'assistance respiratoire au patient, dans lequel le circuit de gaz communiquant avec le patient en utilisant chacun des FiO2 quantité, la haute et basse pression, et le temps haut et bas ;
dans lequel le module de commande est configuré pour communiquer avec le tableau de capteurs pour mesurer des éléments de tableau de données réelles d'un patient d'au moins l'un parmi (i) une quantité de SpO2 de patient, (ii) une quantité d'etCO2 de patient, (iii) un débit expiratoire de pointe, (iv) un volume pulmonaire expiratoire final et (v) une fréquence respiratoire spontanée ;
dans lequel le module de commande comprend un module de configuration initiale (100) avec un mode d'évaluation du volume pulmonaire expiratoire final optimal (150) configuré pour surveiller un certain nombre de paramètres clés du patient afin de déterminer et de calculer périodiquement un volume pulmonaire expiratoire final optimal, un module d'ajustement et de maintenance (200) avec un mode d'oxygénation (300), un mode de recrutement (400) et un mode de ventilation (500), et un module de sevrage (250), et
dans lequel le module de commande est configuré pour comparer le tableau de données réelles du patient au au moins un tableau de données de patient modèle et calculer au moins l'une d'une valeur cible SpO2, d'une valeur cible etCO2 et du volume pulmonaire expiratoire final optimal, laquelle valeur cible SpO2, la valeur cible etCO2 et le volume pulmonaire expiratoire final optimal sont utilisés pour déterminer si le patient doit être initialement sevré, subir un recrutement et une oxygénation accrue, ou être maintenu dans un état de ventilation inchangé pour atteindre la valeur cible SpO2, la valeur cible etCO2, et le volume pulmonaire expiratoire final optimal.

2. Le système de ventilation selon la revendication 1, dans lequel le module de sevrage (250) comprend un protocole de sevrage initial (600), un mode de protocole de ventilation de libération de pression des voies aériennes (700) et un mode de protocole de pression continue des voies aériennes ou CPAP (800).

3. Le système de ventilation selon la revendication 1, dans lequel le au moins un élément de tableau de données de patient modèle comprend en outre au moins l'un parmi une pression positive expiratoire final, une quantité SpO2, une quantité etCO2, un incrément de pression, un incrément de temps, un volume courant, une fréquence respiratoire machine, une pente pression-volume, une pression de déclenchement et une pression d'occlusion.

4. Le système de ventilation selon la revendication 1, dans lequel si la valeur cible de SpO2 est fausse, le module de commande est configuré pour communiquer avec le réseau de capteurs et vérifier le tableau de données réelles du patient pour déterminer la valeur de haute pression ; et
dans lequel, lorsque la valeur haute pression est vraie, le module de commande est configuré pour déterminer une valeur cible de FiO2 qui (a) lorsqu'elle est vraie, le module de commande ajuste au moins l'une de la pompe d'approvisionnement et de la pluralité de vannes pour augmenter la haute pression d'une pression incrément et pour augmenter le temps haut d'un incrément de temps, et (b) lorsqu'il est faux, le module de commande ajuste au moins l'une de la pompe d'approvisionnement et de la pluralité de vannes pour augmenter la quantité de FiO2.

5. Le système de ventilation selon la revendication 1, dans lequel si la valeur cible de SpO2 est vraie, le module de commande est configuré pour communiquer avec le réseau de capteurs et vérifier le tableau de données réelles du patient pour déterminer une valeur cible de FiO2 ; et
dans lequel lorsque la valeur cible de FiO2 (a) est vraie, le module de commande est configuré pour commander au module de commande d'ajuster au moins l'une de la pompe d'approvisionnement et de la pluralité de vannes pour diminuer la quantité de FiO2, et (b) lorsqu'elle est fausse, le module de commande est configuré pour définir une valeur de sevrage initiale comme vraie.

6. Le système de ventilation selon la revendication 1, dans lequel si la valeur cible de SpO2 est fausse, le module de commande est configuré pour communiquer avec le réseau de capteurs et déterminer le tableau de données réelles du patient pour calculer une valeur de recrutement ; et
dans lequel lorsque la valeur de recrutement (i) est vraie, le module de commande est configuré pour générer un signal d'alarme de clinicien, et, (ii) est faux, le module de commande est configuré pour régler au moins l'une de la pompe d'approvisionnement et de la pluralité de vannes pour (a) augmenter la haute pression d'un incrément de pression, (b) augmenter le temps haut d'un incrément de temps, et (c) ajuster le temps bas d'un autre incrément de temps.

7. Le système de ventilation selon la revendication 1, dans lequel si la valeur cible de SpO2 est fausse, le module de commande est configuré pour communiquer avec le réseau de capteurs et déterminer le tableau de données réelles du patient pour calculer une valeur optimale de volume pulmonaire expiratoire final ; et
dans lequel si la valeur optimale du volume pulmonaire expiratoire final (a) est vraie, le module de commande est configuré pour définir une valeur d'oxygénation comme vraie, et (b) si elle est fausse, le module de commande est configuré pour (i) interroger le réseau de capteurs pour mesurer le débit expiratoire de pointe, mesurer la troncature du débit de gaz et calculer un angle de décélération du débit de gaz pour sélectionner l'un des incréments de temps, (ii) définir une valeur de recrutement comme vraie, et (iii) commander la commande module pour régler au moins l'une de la pompe d'approvisionnement et de la pluralité de vannes pour diminuer le temps bas en appliquant l'incrément de temps sélectionné.

8. Le système de ventilation selon la revendication 3, dans lequel le module de commande est configuré pour déterminer la haute pression si la valeur cible etCO2, la comparaison entre la fréquence spontanée et la fréquence respiratoire machine et le temps haut est fausse ; et
dans lequel le module de commande est configuré pour commander au module de commande de régler au moins l'une de la pompe d'approvisionnement et de la pluralité de vannes en augmentant au moins l'un du temps haut et de la haute pression d'au moins un incrément de temps et un incrément de pression respectifs si la haute pression est déterminée comme étant fausse, et dans lequel le module de commande est configuré pour commander au module de commande de régler au moins l'une de la pompe d'approvisionnement et de la pluralité de vannes en augmentant au moins l'un du temps haut d'au moins un incrément de temps respectif si la haute pression est déterminée comme étant vraie.

9. Le système de ventilation selon la revendication 3, dans lequel le module de commande est configuré pour déterminer la haute pression si la valeur cible etCO2 est fausse, la comparaison entre la fréquence spontanée et la fréquence respiratoire de la machine est vraie et le temps haut est vrai ; et
dans lequel le module de commande est configuré pour commander au module de commande de définir une valeur de recrutement comme vraie et d'ajuster au moins l'une de la pompe d'approvisionnement et de la pluralité de vannes en modifiant le temps haut d'au moins un incrément de temps respectif si la haute pression est déterminé comme étant vrai, et dans lequel le module de commande est configuré pour commander au module de commande de régler au moins l'une de la pompe d'approvisionnement et de la pluralité de vannes en diminuant le temps haut et en augmentant la haute pression d'au moins un incrément de temps respectif et incrément de pression si la haute pression est déterminée comme étant fausse.

10. Le système de ventilation selon la revendication 3, dans lequel le module de commande est configuré de telle sorte que lorsque le module de commande détecte que la valeur cible etCO2 est vraie et que le module de commande échantillonne pour déterminer une fréquence spontanée :
a) lorsque la fréquence spontanée est fausse, le module de commande détermine une valeur de tachypnée qui, si elle est vraie, permet au module de commande de définir une valeur de ventilation comme vraie, et
b) lorsque la fréquence spontanée est vraie, le module de commande constate une valeur d'apnée qui (i) lorsqu'elle est vraie permet au module de commande de régler la valeur de ventilation à vraie et (ii) lorsqu'elle est fausse, permet au module de commande de régler une libération de pression des voies aériennes la valeur de sevrage de la ventilation est vraie.

11. Le système de ventilation selon la revendication 3, dans lequel le module de commande est configuré de telle sorte que lorsque le module de commande détecte que la valeur cible etCO2 est vraie et que le module de commande échantillonne la haute pression et définit une valeur de haute pression et échantillonne la fréquence spontanée, et lorsque le module de commande détecte la fréquence spontanée et la valeur haute pression sont vraies, le module de commande initie un sevrage en commandant au module de commande de régler au moins l'une de la pompe d'approvisionnement et de la pluralité de vannes pour diminuer la haute pression d'un incrément de pression et pour augmenter le temps haut d'un incrément de temps.

12. Le système de ventilation selon la revendication 3, comprenant en outre :
le au moins un tableau de données de patient modèle comprenant en outre des critères d'échec de sevrage prédéterminés qui établissent un seuil de FiO2, un seuil de SpO2, un volume courant spontané, une quantité de ventilation minute et une pression d'occlusion des voies respiratoires ;
dans lequel le module de commande est configuré pour communiquer avec le circuit de données pour échantillonner le réseau de capteurs et mesurer au moins l'un des éléments du tableau de données réelles du patient et pour comparer les éléments aux critères d'échec de sevrage prédéterminés pour générer une valeur d'échec de sevrage ; et
dans lequel le module de commande est configuré de telle sorte que lorsque le module de commande détermine (a) que la valeur d'échec de sevrage est vraie, le module de commande commande au module de commande de régler au moins l'une de la pompe d'approvisionnement et de la pluralité de vannes pour augmenter la haute pression par un incrément de pression et pour diminuer le temps haut d'un incrément de temps, et de telle sorte que lorsque le module de commande détermine (b) que la valeur d'échec du sevrage est fausse, le module de commande lance à plusieurs reprises le sevrage cyclique en ordonnant au module de commande d'ajuster au moins l'une de la pompe d'approvisionnement et de la pluralité de vannes pour diminuer la haute pression et le temps élevé par un incrément de pression et de temps.

13. Le système de ventilation selon la revendication 12, dans lequel le module de commande est configuré de telle sorte qu'à chaque fois que le module de commande lance un autre sevrage cyclique, le module de commande constate la haute pression jusqu'à ce qu'un seuil de pression positive continue des voies aériennes soit atteint pour permettre au module de commande de régler une la valeur de pression positive continue des voies aériennes est vraie.

14. Le système de ventilation selon la revendication 3, comprenant en outre :
l'au moins un tableau de données de patient modèle comprenant en outre une pression positive continue prédéterminée des voies respiratoires et des critères d'échec de sevrage prédéterminés établissant un seuil de FiO2, un seuil de SpO2, un volume courant spontané, une quantité de ventilation minute et une pression d'occlusion des voies respiratoires ;
dans lequel le module de commande est configuré pour communiquer avec le circuit de données pour échantillonner le réseau de capteurs et mesurer au moins l'un des éléments du tableau de données réelles du patient et pour comparer les éléments aux critères d'échec de sevrage prédéterminés pour générer une valeur d'échec de sevrage; et
dans lequel le module de commande est configuré de telle sorte que lorsque le module de commande détermine (a) que la valeur d'échec de sevrage est vraie, le module de commande commande au module de commande de régler au moins l'une de la pompe d'approvisionnement et de la pluralité de vannes pour augmenter le positif continu pression des voies aériennes, et de telle sorte que lorsque le module de commande détermine (b) que la valeur d'échec du sevrage est fausse, le module de commande diminue périodiquement la pression positive continue des voies aériennes jusqu'à ce qu'une pression seuil d'extubation soit atteinte.

15. Système de ventilation selon la revendication 14,
dans lequel le module de commande est configuré de telle sorte que lorsque la haute pression est (a) fausse, le module de commande commande au module de commande d'ajuster au moins l'une de la pompe d'approvisionnement et de la pluralité de vannes pour ajuster la pression positive continue des voies aériennes sur la base de la haute pression, et de telle sorte que lorsque la haute pression est (b) vraie, le module de commande commande au module de commande d'ajuster au moins l'une de la pompe d'approvisionnement et de la pluralité de vannes pour s'ajuster à la libération de la pression des voies aériennes ventilation sevrage.
